(19) Europäisches Patentamt
European Patent Office
Office européen des brevets

(11) EP 4 269 448 A1

(12) EUROPEAN PATENT APPLICATION
published in accordance with Art. 153(4) EPC

(43) Date of publication:
01.11.2023 Bulletin 2023/44

(21) Application number: 21910899.0

(22) Date of filing: 22.12.2021

(51) International Patent Classification (IPC):
C08B 15/02 (2006.01)    C08B 15/04 (2006.01)
A61Q 1/00 (2006.01)    A61Q 19/00 (2006.01)
D21H 11/20 (2006.01)    A61K 8/73 (2006.01)
A61K 47/38 (2006.01)

(52) Cooperative Patent Classification (CPC):
A61K 8/73; A61K 47/38; A61Q 1/00; A61Q 19/00;
C08B 15/02; C08B 15/04; D21H 11/20

(86) International application number:
PCT/JP2021/047683

(87) International publication number:
WO 2022/138759 (30.06.2022 Gazette 2022/26)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 24.12.2020 JP 2020215842
18.02.2021 JP 2021024322

(71) Applicant: Toagosei Co., Ltd.
Tokyo, 105-0003 (JP)

(72) Inventors:
• MATSUKI, Shiroshi
Nagoya-shi, Aichi 455-0026 (JP)

• TSUIKI, Toshihiko
Nagoya-shi, Aichi 455-0026 (JP)
• KAYANO, Hidenari
Nagoya-shi, Aichi 455-0026 (JP)
• YOSHIKAWA, Hayato
Nagoya-shi, Aichi 455-0026 (JP)
• KAMIYA, Daisuke
Nagoya-shi, Aichi 455-0026 (JP)

(74) Representative: dompatent von Kreisler Selting
Werner -
Partnerschaft von Patent- und Rechtsanwälten
mbB
Deichmannhaus am Dom
Bahnhofsvorplatz 1
50667 Köln (DE)

(54) PRODUCTION METHOD FOR OXIDIZED CELLULOSE AND NANOCELLULOSE

(57) An object of the present invention is to stably and efficiently provide an oxidized cellulose having excellent fibrillatability. The above object can be achieved by a method of producing an oxidized cellulose, which contains an oxide of a cellulose raw material by a hypochlorous acid or a salt thereof, is substantially free of N-oxyl compounds, and has a degree of polymerization of 600 or less, the method including a step of obtaining an oxidized cellulose by oxidizing a cellulose raw material using a hypochlorous acid or a salt thereof, wherein viscosity of a slurry of the cellulose raw material having the same concentration as when the oxidation is performed is in a range of 1,000 Pa·s or less at 30°C or 40°C.

[FIG. 1]

EP 4 269 448 A1

## Description

### Technical Field

The present invention relates to a method of producing oxidized cellulose and nanocellulose.

### Background Art

Various technologies for producing nanocellulose materials such as cellulose nanofibers (hereinafter referred to as "CNF") by oxidizing various cellulose raw materials with an oxidant and refining the obtained oxidized cellulose have been proposed (for example, refer to Patent Document 1 and Patent Document 2).

Patent Document 1 discloses obtaining oxidized cellulose fibers by oxidizing a cellulose raw material with a hypochlorous acid or a salt thereof as an oxidant under a high concentration condition in which the available chlorine concentration in a reaction system is 14 to 43 mass%. Patent Document 2 discloses obtaining oxidized cellulose fibers by oxidizing a cellulose raw material using a hypochlorous acid or a salt thereof as an oxidant, setting the available chlorine concentration in a reaction system to 6 to 14 mass%, and adjusting the pH to 5.0 to 14.0. In these technologies, since the oxidation treatment is performed without using N-oxyl compounds such as 2,2,6,6-tetramethyl-1-piperidine-N-oxy radical (TEMPO) as a catalyst, N-oxyl compounds do not remain in the cellulose fibers, and thus it is possible to produce nanocellulose materials while reducing the impact on the environment and the like.

Citation List

Patent Document

Patent Document 1: WO 2018/230354

Patent Document 2: WO 2020/027307

### Summary

Technical Problem

<Problem to be solved by first invention (first problem)>

Patent Document 1 and Patent Document 2 disclose, as specific examples of producing nanocellulose materials by refining oxidized cellulose, examples of obtaining nanocellulose materials through a fibrillation step according to a mechanical treatment using an ultrasonic homogenizer. However, the above treatment has room for further improvement in terms of energy required for fibrillation. In the production of nanocellulose materials, in consideration of production cost, there is a demand for oxidized cellulose having ease-of-fibrillatability with which the oxidized cellulose can be fibrillated under mild treatment conditions. In addition, in order to stably produce the refined cellulose fibers or in order to obtain a highly transparent nanocellulose material with less light scattering in a dispersion medium, the fibrillatability of oxidized cellulose before a nanocellulose material is fibrillated is required to be favorable.

Patent Document 1 and Patent Document 2 specifically describe obtaining oxidized cellulose and nanocellulose materials by using several hundred milligrams of a cellulose raw material and reacting a hypochlorous acid or a salt thereof having an available chlorine concentration of 6 to 43 mass% in the reaction system. However, with such methods, the amounts of desired oxidized cellulose and nanocellulose materials are limited, hence a method, in which oxidized cellulose fibers having excellent fibrillatability can be stably and efficiently supplied, is required.

The present invention has been made in view of the above circumstances and a main object of the present invention is to stably and efficiently provide oxidized cellulose having excellent fibrillatability.

<Problem to be solved by second invention (second problem)>

Patent Document 1 and Patent Document 2 disclose, as specific examples of obtaining oxidized cellulose by oxidizing a cellulose raw material using a hypochlorous acid or a salt thereof, examples of obtaining oxidized cellulose by performing solid-liquid separation through filtration of cellulose-based oxides obtained by oxidizing a cellulose raw material using a hypochlorous acid or a salt thereof. However, in the method of producing oxidized cellulose, the solid-liquid separation treatment has room for further improvement in terms of the yield of the obtained oxidized cellulose.

The present invention has been made in view of the above circumstances and a main object of the present invention is to provide a method of producing oxidized cellulose at a high yield.

Solution to Problem

<Means for solving first problem>

The inventors conducted extensive studies and as a result, found that, when a cellulose raw material is oxidized using a hypochlorous acid or a salt thereof and the viscosity of a slurry of the cellulose raw material having the same concentration as when the oxidation is performed is controlled to be within a range of 1,000 Pa·s or less, oxidized cellulose having excellent fibrillatability can be stably and efficiently produced, and completed the present invention.

<Means for solving second problem>

The inventors conducted extensive studies and as a result, found that, when the method further includes a step of obtaining oxidized cellulose by solid-liquid separation of an oxide dispersion containing a cellulose-based oxide and a dispersion medium, with the pH of the oxide dispersion being 4.0 or less and the oxide dispersion being substantially free of N-oxyl compounds, or the method further includes a step of obtaining the cellulose-based oxide by oxidizing a cellulose raw material using a predetermined amount range of a hypochlorous acid or a salt thereof, with the pH of the oxide dispersion being 4.0 or less, the oxidized cellulose can thus be obtained at a high yield, and completed the present invention.

The present invention is specifically as follows.

[1] A method of producing an oxidized cellulose, which contains an oxide of a cellulose raw material by a hypochlorous acid or a salt thereof, is substantially free of N-oxyl compounds, and has a degree of polymerization of 600 or less, the method including

a step of obtaining an oxidized cellulose by oxidizing a cellulose raw material using a hypochlorous acid or a salt thereof,

wherein a viscosity of a slurry of the cellulose raw material having the same concentration as when the oxidation is performed, which is measured using a viscometer including an SPP rotor under measurement conditions of a rotational speed of 100 rpm and 30°C or 40°C, is in a range of 1,000 Pa·s or less.

[2] The production method according to [1],
wherein a concentration of the cellulose raw material with respect to a total amount of a reaction mixture is 35 mass% or less.
[3] The production method according to [1] or [2],
wherein the concentration of the cellulose raw material with respect to the total amount of the reaction mixture is more than 6.5 mass%.
[4] The production method according to any one of [1] to [3],
wherein an available chlorine concentration of the hypochlorous acid or the salt thereof in a reaction system is 6 mass% or more and 43 mass% or less.
[5] The production method according to any one of [1] to [3],
wherein an available chlorine concentration of the hypochlorous acid or the salt thereof in a reaction system is less than 14 mass%.
[6] The production method according to any one of [1] to [5],
wherein a reaction temperature for the oxidation is 30°C or higher.
[7] The production method according to any one of [1] to [6],
wherein a reaction time for the oxidation is 2 hours or longer.
[8] The production method according to any one of [1] to [7],
wherein pH of the reaction system is less than 11.
[9] The production method according to any one of [1] to [8],
wherein the viscosity is in a range of 30 Pa·s or less.
[10] A method of producing a nanocellulose, the method including
a step of obtaining the nanocellulose through fibrillation after the step of oxidizing in the production method according to any one of [1] to [9].

[11] A method of producing an oxidized cellulose, the method including

a step of obtaining the oxidized cellulose by solid-liquid separation of an oxide dispersion containing a cellulose-based oxide and a dispersion medium,
wherein pH of the oxide dispersion is 4.0 or less, and the oxide dispersion is substantially free of N-oxyl compounds.

[12] The production method according to [11], further including
a step of obtaining the cellulose-based oxide by oxidizing a cellulose raw material using a hypochlorous acid or a salt thereof.
[13] A method of producing an oxidized cellulose, the method including:

A step of obtaining a cellulose-based oxide by oxidizing a cellulose raw material using a hypochlorous acid or a salt thereof with a mass proportion of 0.2 or more with respect to the cellulose raw material; and
a step of obtaining the oxidized cellulose by solid-liquid separation of an oxide dispersion containing the cellulose-based oxide and a dispersion medium,
wherein pH of the oxide dispersion is 4.0 or less.

[14] The production method according to [12] or [13], further including
a step of treating the hypochlorous acid or the salt thereof in the oxide dispersion.
[15] The production method according to any one of [11] to [14], further including
a step of adding an acid and/or a cation ion exchange resin to prepare the oxide dispersion having pH of 4.0 or less.
[16] The production method according to any one of [11] to [15], further including
a step of adding a base to adjust pH of an oxidized cellulose dispersion containing the oxidized cellulose and a dispersion medium to more than 4.0.
[17] The production method according to any one of [11] to [16],
wherein the pH of the oxide dispersion is 2.5 or less.
[18] The production method according to any one of [11] to [17],
wherein the step of obtaining the oxidized cellulose is a step of solid-liquid separation by filtering the oxide dispersion.
[19] The production method according to any one of [11] to [18], further including
a step of washing the oxide dispersion or the oxidized cellulose with an acidic washing solution.
[20] The production method according to any one of [11] to [19],
wherein a degree of polymerization of the oxidized cellulose is 600 or less.
[21] Oxidized cellulose obtained by the production method according to any one of [11] to [20].
[22] A method of producing a nanocellulose, the method including
a step of obtaining the nanocellulose by fibrillating the oxidized cellulose obtained by the production method according to any one of [11] to [20].
[23] Nanocellulose obtained by the production method according to [22].

Advantageous Effects of Invention

<Effects of first invention>

According to a production method of the present invention, it is possible to stably and efficiently obtain oxidized cellulose having excellent fibrillatability. Particularly, oxidized cellulose according to the present invention can be uniformly refined even when a fibrillation treatment is performed under mild conditions, and has excellent ease-of-fibrillatability.

<Effects of second invention>

According to a method of producing oxidized cellulose of the present invention, the oxidized cellulose can be obtained at a high yield. Particularly, the production method of the present invention has excellent production efficiency because clogging can be reduced when solid-liquid separation is performed through filtration.

**Brief Description of Drawings**

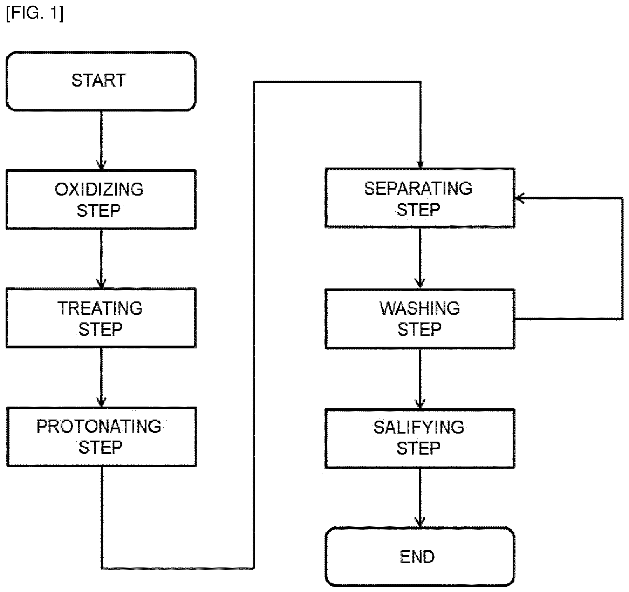

Fig. 1 is a flowchart showing an example of a production method of a second invention.

**Description of Embodiments**

The first invention mainly focuses on obtaining oxidized cellulose by oxidizing a cellulose raw material. The second invention mainly focuses on a post-treatment of oxidized cellulose obtained by oxidizing a cellulose raw material. Hereinafter, the first invention and the second invention will be described separately for respective items, but the specific embodiment of the first invention may be referred to in the second invention, and the specific embodiment of the second invention may be referred to in the first invention. In addition, the first invention and the second invention may be combined to provide a method including a step of obtaining oxidized cellulose by oxidizing a cellulose raw material and a step of post-treating the oxidized cellulose.

<<Embodiment for performing first invention>>

<Method of producing oxidized cellulose>

The production method of the present invention is a method of producing oxidized cellulose, which contains an oxide of a cellulose raw material by a hypochlorous acid or a salt thereof, is substantially free of N-oxyl compounds, and has a degree of polymerization of 600 or less. The production method of the present invention includes a step of obtaining oxidized cellulose by oxidizing a cellulose raw material using a hypochlorous acid or a salt thereof. In addition, the viscosity of a slurry of the cellulose raw material having the same concentration as when the oxidation is performed, which is measured using a viscometer including an SPP rotor under measurement conditions of a rotational speed of 100 rpm and 30°C or 40°C (hereinafter referred to as "measurement conditions A") is in a range of 1,000 Pa·s or less.
The production method of the present invention can also be rephrased as follows.
A method of producing oxidized cellulose, which contains an oxide of a cellulose raw material by a hypochlorous acid or a salt thereof, is substantially free of N-oxyl compounds, and has a degree of polymerization of 600 or less, including

a step of obtaining oxidized cellulose by oxidizing a cellulose raw material in a reaction system containing a hypochlorous acid or a salt thereof and the cellulose raw material,

wherein the reaction system contains the cellulose raw material at a predetermined concentration, and

wherein the predetermined concentration is a concentration at which, when a slurry composed of only the cellulose raw material and water, and containing the cellulose raw material at the predetermined concentration is prepared, its viscosity measured under measurement conditions A is 1,000 Pa·s or less.

The production method of the present invention can also be rephrased as follows. Here, the "initial viscosity of the reaction system" below is the viscosity of the reaction system when the reaction starts.
A method of producing oxidized cellulose, which contains an oxide of a cellulose raw material by a hypochlorous acid or a salt thereof, is substantially free of N-oxyl compounds, and has a degree of polymerization of 600 or less, including

a step of obtaining oxidized cellulose by oxidizing a cellulose raw material in a reaction system containing a hypochlorous acid or a salt thereof and the cellulose raw material,

wherein the initial viscosity of the reaction system is 1,000 Pa·s or less under measurement conditions A.

The time at which the reaction starts is preferably within 10 minutes, more preferably within 5 minutes, and still more preferably within 1 minute after components constituting the reaction system have been completely added to the system. As one specific aspect, the time at which the reaction starts is preferably within 10 minutes, more preferably within 5 minutes, and still more preferably within 1 minute after the cellulose raw material has been completely added to the system containing the hypochlorous acid or salt thereof.
In the conventional method of producing oxidized cellulose, there are problems that the amount of raw material cellulose to be subjected to the oxidation reaction is small, the amount of the obtained oxidized cellulose is limited, and the productivity of desired oxidized cellulose cannot be improved. One method of improving the productivity of oxidized cellulose is a method of increasing the scale of the reaction and increasing the concentration of the cellulose raw material in the reaction system. As a result of studies by the inventors, it has been found that, in order to obtain oxidized cellulose having excellent fibrillatability, it is desirable to sufficiently supply the hypochlorous acid or salt thereof to the surface of the raw material cellulose, and if the amount of raw material cellulose is increased, a part in which the hypochlorous acid or salt thereof cannot sufficiently act on the raw material cellulose occurs, and desired oxidized cellulose cannot be obtained satisfactorily. The inventors found that, by controlling the viscosity of the reaction system, specifically, by performing oxidation at such a concentration of the cellulose raw material that if a slurry composed of only the cellulose raw material and water was prepared in which the concentration of the cellulose raw material is the same as the concentration when the oxidation is performed (that is, the concentration of the cellulose raw material used in the preparation for the oxidation reaction) and the viscosity thereof (hereinafter referred to as a slurry viscosity) was measured, the viscosity was 1,000 Pa·s or less at 30°C or 40°C, the hypochlorous acid or the salt thereof becomes sufficiently uniform in the reaction system, and oxidized cellulose having excellent fibrillatability can be stably and efficiently produced, and completed the present invention. In addition, when the concentration of the cellulose raw material in the reaction system is set so that the viscosity is 1,000 Pa·s or less, the reaction system can be uniformized using a stirrer or kneader to be described below.

The reaction system in the present invention is a mixture (hereinafter referred to as a reaction mixture) of constituent components (including a dispersion medium) during an oxidation reaction.

In order to facilitate a stirring or kneading operation, the slurry viscosity or the initial viscosity of the reaction system in the present invention is preferably 30 Pa·s or less, more preferably 20 Pa·s or less, still more preferably 10 Pa·s or less, and yet more preferably 5 Pa·s or less.

A smaller lower limit value of the slurry viscosity or the initial viscosity of the reaction system is more preferable in consideration of uniformity of the reaction system. The lower limit value is not particularly limited, and may be more than 0 Pa·s, 0.01 Pa·s or more, 0.1 Pa·s or more, or 0.3 Pa·s or more.

The range of the slurry viscosity or the initial viscosity of the reaction system may be, for example, more than 0 Pa·s and 30 Pa·s or less, 0.01 Pa·s or more and 20 Pa·s or less, 0.1 Pa·s or more and 10 Pa·s or less, or 0.3 Pa·s or more and 5 Pa·s or less.

Methods of controlling the slurry viscosity or the initial viscosity of the reaction system include, for example, a method of adjusting the concentration of the cellulose raw material and the temperature. Specifically, control is performed such that the slurry viscosity or the initial viscosity of the reaction system increases as the concentration of the cellulose raw material increases. In addition, control is performed such that the slurry viscosity or the initial viscosity of the reaction system increases as the temperature during oxidation increases.

**The** slurry viscosity is measured using a slurry of the cellulose raw material. The viscosity measured from the slurry reproduces the initial viscosity of the reaction system (mixture of constituent components during the oxidation reaction). The slurry viscosity is measured using a viscometer including an SPP rotor at 30°C or 40°C when a slurry of the cellulose raw material having the same concentration as when the oxidation is performed is prepared, and stirred at a rotational speed of 100 rpm. Specifically, the initial viscosity of the reaction system can be measured by the method described in examples.

As the oxidation of the cellulose raw material proceeds and oxidized cellulose is produced, the viscosity of the reaction system tends to decrease. The viscosity of the reaction system tends to be the highest when the reaction starts. It can be said that the slurry viscosity is the same as the viscosity of the reaction system when the reaction starts (that is, the initial viscosity of the reaction system).

The cellulose raw material used in the present invention is not particularly limited as long as it is a material mainly composed of cellulose, and examples thereof include pulp, natural cellulose, regenerated cellulose, and fine cellulose depolymerized by mechanically treating cellulose. As the cellulose raw material, commercial products such as crystal cellulose made from pulp can be used without change. In addition, unused biomass containing a large amount of cellulose components such as bean curd leftovers and soybean hulls may be used as a raw material. As the raw material, cotton and sea squirt can be used.

The type of pulp is not particularly limited, and examples thereof include coniferous trees and broadleaf tree, bamboo, straw, bagasse, cannabis, kenaf in addition to coniferous trees. These pulps may be used alone or two or more thereof may be used in combination. In order to facilitate permeation of an oxidant to be used into the raw material pulp, the cellulose raw material may be treated with an alkali having an appropriate concentration in advance.

In addition, as the pulp, for example, mechanical pulp, chemical mechanical pulp, semi-chemical pulp, or chemical pulp (kraft pulp, sulfite pulp, and alkaline pulp) can be used.

In the production method of the present invention, as the cellulose raw material, fine cellulose obtained by mechanically treating or chemically treating cellulose may be used. As the fine cellulose, powdered pulp can be suitably used. When powdered pulp is used, there is a tendency for refining to further proceed and nanocellulose can be efficiently obtained. In addition, the particle size of the powdered pulp is generally in a range of 1 to 1,000 μm, preferably in a range of 1 to 500 μm, and more preferably in a range of 1 to 100 μm. The particle size referred to here is the average particle size, and is a value when a cumulative volume distribution is 50% if a particle size distribution is expressed as a cumulative volume distribution using a laser scattering method as a measurement principle.

The range of the degree of crystallization of the cellulose raw material is not limited as long as it is within the range in which nanocellulose can be obtained, and is generally a range of 10 to 90%. The degree of crystallization is preferably in a range of 20 to 80% and more preferably in a range of 30 to 70%.

The degree of crystallization can be calculated from the ratio between a crystal part and non-crystal part by

performing solid $^{13}$C-NMR measurement on the cellulose raw material. The degree of crystallization can be specifically calculated by the method described in examples.

Examples of the hypochlorous acid or salt thereof used for oxidizing the cellulose raw material include hypochlorous acid water, sodium hypochlorite, potassium hypochlorite, calcium hypochlorite, and ammonium hypochlorite. Among these, sodium hypochlorite is preferable in consideration of ease of handling.

Examples of methods of producing oxidized cellulose by oxidizing the cellulose raw material include a method of mixing a cellulose raw material and a reaction solution containing a hypochlorous acid or a salt thereof. The solvent contained in the reaction solution is preferably water because it is easy to handle and side reactions are unlikely to occur.

In oxidation, the amount of the hypochlorous acid or salt thereof used is not particularly limited, and it is preferable to use a hypochlorous acid or a salt thereof having an available chlorine concentration of 6 mass% or more and 43 mass% or less. When the hypochlorous acid or salt thereof having an available chlorine concentration of 6 mass% or more and 43 mass% or less is used, the carboxy group content in the oxidized cellulose can be sufficiently increased, refining sufficiently proceeds, and a mechanical fibrillation treatment after the oxidation reaction can be omitted.

In addition, the available chlorine concentration of the hypochlorous acid or salt thereof in the reaction solution (reaction system) is preferably in a range of 6 to 43 mass%.

In order to allow refining of the oxidized cellulose to proceed more smoothly, the lower limit value of the available chlorine concentration is more preferably 7 mass% or more, still more preferably 8 mass% or more, yet more preferably 8.5 mass% or more, and yet more preferably 9 mass% or more. In addition, in order to prevent excessive decomposition of cellulose, the available chlorine concentration of the reaction solution is more preferably 40 mass% or less, and still more preferably 38 mass% or less. Regarding the range of the available chlorine concentration of the reaction solution, the above lower limits and upper limits can be appropriately combined. The range of the available chlorine concentration is more preferably 7 to 43 mass%, and more preferably 8 to 43 mass%.

In order reduce oxidized cellulose production costs and improve productivity such as availability and ease of handling of the hypochlorous acid or salt thereof, it is preferable that the available chlorine concentration be kept low. In this regard, the upper limit value of the available chlorine concentration is preferably less than 14 mass%, more preferably 13 mass% or less, still more preferably 12 mass% or less, and yet more preferably 11 mass% or less. In order to allow refining of the oxidized cellulose to proceed more smoothly and improve productivity, the range of the available chlorine concentration is preferably 6 mass% or more and less than 14 mass%, more preferably 7 mass% or more and less than 14 mass%, still more preferably 7 mass% or more and 13 mass% or less, and yet more preferably 8 mass% or more and 13 mass% or less.

Here, the available chlorine concentration of the hypochlorous acid or salt thereof is defined as follows. The hypochlorous acid is a weak acid that exists in an aqueous solution, and hypochlorites are compounds in which a hydrogen ion of the hypochlorous acid is substituted with another cation. For example, since sodium hypochlorite, which is a hypochlorite, exists in a solvent (preferably in an aqueous solution), the concentration is measured as the amount of available chlorine in the solution rather than the concentration of sodium hypochlorite. Here, regarding available chlorine in sodium hypochlorite, oxidizing power of divalent oxygen atoms generated by decomposition of sodium hypochlorite corresponds to 2-atom equivalents of monovalent chlorine, and thus the bound chlorine atom of sodium hypochlorite (NaClO) has the same oxidizing power as two atoms of unbound chlorine ($Cl_2$), and the available chlorine=2×(chlorine in NaClO). As a specific measurement procedure, first, a sample is accurately weighed, water, potassium iodide and acetic acid are added, the mixture is left, the released iodine is titrated with a sodium thiosulfate solution using an aqueous starch solution as an indicator, and the available chlorine concentration is measured.

In the reaction in the production method of the present invention, adjustment of pH and the range of pH are arbitrary, but it is preferable to adjust the pH to a range of 5.0 or more. Within this range, the oxidation reaction of the cellulose raw material can sufficiently proceed, the carboxy group content in the oxidized cellulose sufficiently increases, and refining by stirring tends to proceed easily. The pH of the reaction system is more preferably 7.0 or more, still more preferably 8.0 or more, yet more preferably 8.5 or more, yet more preferably 9.0 or more, and most preferably 9.5 or more. The upper limit of the pH of the reaction system is not particularly limited, and is preferably 14.5 or less, more preferably 14.0 or less, still more preferably 13.0 or less, yet more preferably 12.5 or less, yet more preferably 12.0 or less, and most preferably 11.5 or less. In addition, the range of the pH of the reaction system is more preferably 7.0 to 14.0, still more preferably 8.0 to 13.5, and yet more preferably 8.5 to 13.0.

When the scale of the reaction is increased and the concentration of the cellulose raw material is increased, the viscosity of the reaction system increases, and it tends to become difficult to sufficiently supply a hypochlorous acid or a salt thereof to the surface of the raw material cellulose. In addition, as described above, in order to reduce oxidized cellulose production cost and improve productivity such as availability and ease of handling of the hypochlorous acid or salt thereof, it is preferable that the available chlorine concentration be kept low. On the other hand, when the available chlorine concentration is kept low, for example, the available chlorine concentration is set to be less than 14 mass%, the oxidation reaction insufficiently proceeds, which may lead to a decrease in fibrillatability of the oxidized cellulose. In order to reduce the available chlorine concentration and improve the oxidizing action of the hypochlorous acid or salt thereof on the surface of the raw material cellulose, the pH of the reaction system is preferably less than 11, more preferably 10.7 or less, and still more preferably 10.5 or less. In this case, the lower limit of the pH of the reaction system is not particularly limited, and is generally 5.0 or more, preferably 6.0 or more, more preferably 7.0 or more, still more preferably 8.0 or more, yet more preferably 9.0 or more, and yet more preferably more than 9.0. The pH range of the reaction system may be obtained by appropriately combining these upper limit values and lower limit values. The pH of the reaction system is preferably 5.0 or more less than 11, more preferably 6.0 or more less than 11, still more preferably 7.0 or more less than 11, yet more preferably 8.0 or more less than 11, yet more preferably 8.0 or more and 10.7 or less, yet more preferably 9.0 or more and 10.7 or less, yet more preferably 9.0 or more and 10.5 or less, and most preferably more than 9.0 and 10.5 or less.

During the reaction, the pH of the reaction system decreases when carboxy groups are generated in the cellulose raw material due to the oxidation reaction. Therefore, in order to allow the oxidation reaction to proceed efficiently, it is preferable to add an alkaline agent (for example, sodium hydroxide, etc.) or an acid (for example, hydrochloric acid, etc.) to the reaction system and perform the oxidation reaction while adjusting the pH of the reaction system.

Hereinafter, a method of producing oxidized cellulose will be additionally described using a case in which sodium hypochlorite is used as a hypochlorous acid or a salt thereof as an example.

When the cellulose raw material is oxidized using sodium hypochlorite, the reaction solution is preferably an aqueous sodium hypochlorite solution. Examples of a method of adjusting the available chlorine concentration of the aqueous sodium hypochlorite solution to a desired concentration (for example, a desired concentration: a range of 6 mass% to 43 mass%) include a method of concentrating an aqueous sodium hypochlorite solution with an available chlorine concentration lower than a desired concentration, a method of diluting an aqueous sodium hypochlorite solution with an available chlorine concentration higher than a target concentration, and a method of dissolving sodium hypochlorite crystals (for example, sodium hypochlorite pentahydrate) in a solvent. Among these, it is preferable to adjust the available chlorine concentration of an oxidant by the method of diluting an aqueous sodium hypochlorite solution or the method of dissolving sodium hypochlorite crystals in a solvent because self-decomposition is weak (that is, the decrease in the available chlorine concentration is small), and adjustment of the available chlorine concentration is simple.

A method of mixing a cellulose raw material and an aqueous sodium hypochlorite solution is not particularly limited, and in consideration of ease of operation, it is preferable to add the cellulose raw material to the aqueous sodium hypochlorite solution and mix them.

In order to allow the oxidation reaction of the cellulose raw material proceed efficiently, it is preferable to stir a mixed solution containing the cellulose raw material and the aqueous sodium hypochlorite solution during the oxidation reaction. Examples of stirring methods include stirring using a stirrer with a stirring blade, a homomixer, a disper-type mixer, a homogenizer, an external circulation stir and the like. Among these, in order to allow the oxidation reaction of the cellulose raw material proceed smoothly and easily adjust the degree of polymerization of the oxidized cellulose to a predetermined value or less, a method using one, two or more of shear type stirrers such as homomixers and homogenizers, stirrers with a stirring blade, and disper-type mixers is preferable, and a method using a stirrer with a stirring blade is particularly preferable. When a stirrer with a stirring blade is used, a device including known stirring blades such as propeller blades, paddle blades, turbine blades, swept blades, anchor blades, gate blades, Maxblend blades, fullzone blades, helical ribbon blades, and screw blades (with draft tubes, etc.) can be used as the stirrer. In addition, when a stirrer with a stirring blade is used, it is preferable to perform stirring at a rotational speed of 50 to 1,000 rpm. In addition, a single-screw kneader, and a multi-screw kneader such as a twin-screw kneader can also be used.

The reaction temperature in the oxidation reaction may be generally in a range of 15°C to 100°C. In order to further improve the progress of the oxidation reaction, the reaction temperature is preferably 30°C or higher, more preferably higher than 30°C, still more preferably 31°C or higher, and yet more preferably 35°C or higher. If the reaction temperature is higher, the viscosity is higher, and the uniformity of the reaction system tends to decrease. In order to improve the uniformity of the reaction system and improve productivity, the reaction temperature is preferably 60°C or lower, more preferably 55°C or lower, and still more preferably 40°C or lower. The reaction temperature referred to here is a temperature obtained by measuring the temperature of the reaction mixture.

The reaction time of the oxidation reaction can be set according to the degree of oxidation progress, but is generally about 15 minutes to 50 hours. In order to further improve the progress of the oxidation reaction, the reaction time is preferably 2 hours or longer, more preferably longer than 2 hours, and still more preferably 3 hours or longer. The upper limit value of the reaction time is not particularly limited, and is preferably 20 hours or shorter, more preferably 15 hours or shorter, and still more preferably 12 hours or shorter.

In order to set the slurry viscosity or the initial viscosity of the reaction system to be within a range of 1,000 Pa·s or less and improve operability such as facilitating stirring during the oxidation reaction, the concentration of the cellulose raw material with respect to a total amount of the reaction mixture (that is, a total amount of the reaction system) when the oxidation reaction starts is preferably 35 mass% or less, more preferably 20 mass% or less, still more preferably 15 mass% or less, and yet more preferably 10 mass% or less.

The lower limit of the concentration of the cellulose raw material may be generally 0.1 mass% or more, and in order to improve productivity, it is preferably more than 6.5 mass%, more preferably 6.6 mass% or more, still more preferably 6.8 mass% or more, and yet more preferably 7 mass% or more.

The concentration of the cellulose raw material is preferably in a range of more than 6.5 mass% and 35 mass% or less, more preferably in a range of more than 6.5 mass% and 20 mass% or less, still more preferably in a range of more than 6.5 mass% and 15 mass% or less, and yet more preferably in a range of more than 6.5 mass% and 10 mass% or less.

The concentration of the cellulose raw material during the oxidation reaction here is the concentration of the cellulose raw material during preparation.

The pressure when the reaction is performed is not particularly limited, and is generally in a range of normal pressure or more and 1.0 MPaG or less (gauge pressure, hereinafter the same). Here, normal pressure is a pressure level equal to atmospheric pressure.

When oxidation is performed under pressure, the amount of the hypochlorous acid or salt thereof used is reduced, and there is a tendency for oxidized cellulose to be able to be produced more efficiently. In consideration of efficiency, the pressure is preferably 0.1 MPaG or more and 1.0 MPaG or less. In this case, the available chlorine concentration of the hypochlorous acid or salt thereof may be more than 0 mass% and 43 mass% or less, and in order to improve efficiency, it is preferably 0.1 mass% or more and 20 mass% or less, more preferably 1.0 mass% or more and 15 mass% or less, and still more preferably 1.0 mass% or more and 10 mass% or less.

In the production of oxidized cellulose, after the cellulose raw material is oxidized, a treatment for stopping the oxidation reaction may be performed. That is, the method may further include a step of treating a hypochlorous acid or a salt thereof used in the oxidizing step (hereinafter referred to as a "treating step"). A method of treating the hypochlorous acid or salt thereof is not particularly limited, and the hypochlorous acid or salt thereof may be treated by self-decomposition under UV emission or high temperature conditions, but a method of reducing the hypochlorous acid or salt thereof is preferably used. Specifically, a method of adding reducing agents such as sulfites, sulfamic acid or a salt thereof, thiosulfate, hydrogen peroxide, oxalic acid or a salt thereof, formic acid or a salt thereof, or hypophosphite and a method of adding a decomposition catalyst such as nickel oxide may be exemplified.

Examples of sulfites include sulfite, hydrogen sulfite, pyrosulfite, and hyposulfite, and these may be hydrate. Specific examples of sulfites include sodium bisulfite, potassium bisulfite, ammonium bisulfite, calcium bisulfite, sodium sulfite, potassium sulfite, ammonium sulfite, zinc sulfite, ammonium sulfite, sodium hyposulfite, potassium hyposulfite, calcium hyposulfite, sodium pyrosulfite, potassium pyrosulfite, magnesium pyrosulfite, calcium pyrosulfite, and ammonium pyrosulfite, and among these, sodium sulfite is preferable.

Among the above sulfamic acids or salts thereof, sulfamates are preferable. Specific examples of sulfamates include sodium sulfamate, potassium sulfamate, calcium sulfamate, and nickel sulfamate.

Specific examples of thiosulfates include sodium thiosulfate, potassium thiosulfate, and ammonium thiosulfate.

Specific examples of oxalates include sodium oxalate and potassium oxalate.

Specific examples of formates include sodium formate and potassium formate.

Specific examples of hypophosphites include sodium hypophosphite.

These reducing agents may be used alone or two or more thereof may be used in combination.

The amount of the reducing agent added may be appropriately adjusted according to the amount of the hypochlorous acid or salt thereof (available chlorine concentration). In addition, in addition to the method of treating the hypochlorous acid or salt thereof, a method of adding an acid or a metal catalyst to stop the reaction of oxidizing the cellulose raw material may be used in combination.

When a known isolation treatment such as centrifugation and filtration is performed using the solution containing oxidized cellulose obtained according to the reaction, and purification is additionally performed as necessary, it is possible to obtain oxidized cellulose as an oxide of the cellulose raw material with the hypochlorous acid or salt thereof. In addition, the solution containing oxidized cellulose obtained according to the reaction may be directly used in the next step.

[Oxidized cellulose]

In the production method of the present invention, a cellulose raw material is oxidized with a hypochlorous acid or a salt thereof, and thereby oxidized cellulose can be obtained. The oxidized cellulose is preferably in the form of a slurry. The slurry referred to here is a suspension containing oxidized cellulose. The slurry may contain the solvent used during oxidation. In addition, a dispersion medium may be appropriately added to form a slurry. Since the oxidized cellulose is a slurry, it is easy to handle and tends to be easily refined.

If the oxidized cellulose in the present invention is a slurry, the amount of oxidized cellulose with respect to a total amount of 100 mass% of the slurry is generally in a range of 0.1 mass% or more and 95 mass% or less, preferably 1 mass% or more and 50 mass% or less, and more preferably 1 mass% or more and 30 mass% or less.

The oxidized cellulose in the present invention includes fibrous cellulose obtained by oxidizing a cellulose raw material with a hypochlorous acid or a salt thereof. The oxidized cellulose in the present invention is also called an oxidized cellulose fiber. That is, the oxidized cellulose in the present invention includes an oxide of the cellulose raw material by a hypochlorous acid or a salt thereof. Here, the main component of plants is cellulose, and bundles of cellulose molecules are called cellulose microfibrils. Cellulose in the cellulose raw material is also contained in the form of cellulose microfibrils.

(Degree of polymerization)

The degree of polymerization of the oxidized cellulose in the present invention is 600 or less. If the degree of polymerization of the oxidized cellulose is more than 600, a large amount of energy tends to be required for fibrillation, and there is a tendency for sufficient ease-of-fibrillatability not be exhibited. If the degree of polymerization of the oxidized cellulose is 600 or less, the oxidized cellulose is refined under mild conditions and can be refined by general stirring and kneading, and nanocellulose tends to be obtained efficiently. In consideration of ease-of-fibrillatability, the lower limit of the degree of polymerization of the oxidized cellulose is not particularly set. However, if the degree of polymerization of the oxidized cellulose is less than 30, the proportion of particulate cellulose is larger than that of fibrous cellulose, the quality of the slurry containing oxidized cellulose becomes uneven and the viscosity becomes unstable. In view of this, the degree of polymerization of the oxidized cellulose is preferably 30 to 600.

The degree of polymerization is more preferably 580 or less, still more preferably 560 or less, yet more preferably 550 or less, yet more preferably 500 or less, yet more preferably 450 or less, and yet more preferably 400 or less. In order to further improve the viscosity stability of the slurry, the lower limit of the degree of polymerization is more preferably 50 or more, still more preferably 60 or more, yet more preferably 80 or more, yet more preferably 90 or more, yet more preferably 100 or more, and particularly preferably 110 or more. A preferable range of the degree of polymerization can be determined by appropriately combining the above upper limits and lower limits. The degree of polymerization of the oxidized cellulose is more preferably 50 to 600, still more preferably 60 to 600, yet more preferably 80 to 600, still more preferably 80 to 550, still more preferably 80 to 500, still more preferably 80 to 450, and particularly preferably 80 to 400.

When the slurry viscosity or the initial viscosity of the reaction system is set to 1,000 Pa·s or less the degree of polymerization of the oxidized cellulose can be 600 or less because the hypochlorous acid or salt thereof becomes sufficiently uniform in the reaction system.

In addition, the degree of polymerization of the oxidized cellulose can be adjusted, for example, by changing the reaction time, the reaction temperature, and the pH during the oxidation reaction and the available chlorine concentration of the hypochlorous acid or salt thereof and the like. Specifically, since the degree of polymerization tends to decrease when the degree of oxidation increases, in order to reduce the degree of polymerization, for example, methods of increasing the oxidation reaction time and/or reaction temperature may be exemplified.

In addition, the degree of polymerization of the oxidized cellulose can be adjusted according to stirring conditions of the reaction system during the oxidation reaction. For example, under conditions in which the reaction system is sufficiently uniformized using a stirring blade or the like, the oxidation reaction smoothly proceeds, and the degree of polymerization tends to decrease. On the other hand, under conditions in which the reaction system is likely to be insufficiently stirred such as stirring with a stirrer, the reaction tends to be uneven, and it is difficult to sufficiently reduce the degree of polymerization of the oxidized cellulose. In addition, the degree of polymerization of the oxidized cellulose tends to vary depending on the selection of raw material cellulose. Therefore, the degree of polymerization of the oxidized cellulose can be adjusted by selecting the cellulose raw material. Here, in this specification, the degree of polymerization of the oxidized cellulose is the average degree of polymerization (viscosity average degree of polymerization) measured by a viscosity method. Specifically, the degree of polymerization of the oxidized cellulose can be measured by the method described in examples.

(Carboxy group content)

The carboxy group content of the oxidized cellulose is preferably 0.30 to 2.0 mmol/g. If the carboxy group content is 0.30 mmol/g or more, sufficient ease-of-fibrillatability can be imparted to the oxidized cellulose. Thereby, the oxidized cellulose can be refined under mild conditions and tends to be refined by general stirring and kneading. On the other hand, if the carboxy group content is 2.0 mmol/g or less, it is possible to minimize excessive decomposition when oxidized cellulose is mixed with other components and it is possible to obtain nanocellulose with a small proportion of particulate cellulose and uniform quality. In view of this, the carboxy group content of the oxidized cellulose is more preferably 0.35 mmol/g or more, still more preferably 0.40 mmol/g or more, yet more preferably 0.42 mmol/g or more, yet more preferably 0.50 mmol/g or more, yet more preferably more than 0.50 mmol/g, and yet more preferably 0.55 mmol/g or more. The upper limit of the carboxy group content is more preferably 1.5 mmol/g or less, still more preferably 1.2 mmol/g or less, yet more preferably 1.0 mmol/g or less, and yet more preferably 0.9 mmol/g. A preferable range of the carboxy group content can be determined by appropriately combining the above upper limits and lower limits. The carboxy group content of the oxidized cellulose is more preferably 0.35 to 2.0 mmol/g, still more preferably 0.35 to 1.5 mmol/g, yet more preferably 0.40 to 1.5 mmol/g, yet more preferably 0.50 to 1.2 mmol/g, yet more preferably more than 0.50 to 1.2 mmol/g, and yet more preferably 0.55 to 1.0 mmol/g.

Here, the carboxy group content (mmol/g) in oxidized cellulose is a value calculated using the following formula from the amount of sodium hydroxide (a) consumed in the neutralization stage of a weak acid with a mild change in the electrical conductivity, by adding a 0.1 mol/L aqueous hydrochloric acid solution to an aqueous solution in which oxidized cellulose is mixed with water, adjusting the pH to 2.5, then adding a 0.05 N aqueous sodium hydroxide solution dropwise, and measuring the electrical conductivity until the pH reaches 11.0. The carboxy group content of the oxidized cellulose can be adjusted by changing the reaction time, the reaction temperature, and the pH of the reaction solution during the oxidation reaction.

$$\text{carboxy group content} = a \text{ (ml)} \times 0.05 / \text{oxidized cellulose mass (g)}$$

Specifically, the carboxy group content can be measured according to the following procedure.

A 0.1 M aqueous hydrochloric acid solution is added to 60 ml of an oxidized cellulose aqueous dispersion in which the concentration of oxidized cellulose is adjusted to 0.5 mass%, the pH is adjusted to 2.5, a 0.05 N aqueous sodium hydroxide solution is then added dropwise, the electrical conductivity is measured until the pH reaches 11.0, and the carboxy group content (mmol/g) is calculated from the amount of sodium hydroxide (a) consumed in the neutralization stage of a weak acid with a mild change in the electrical conductivity using the above formula.

(Light transmittance)

In the oxidized cellulose in the present invention, the light transmittance of a nanocellulose aqueous dispersion solution obtained by fibrillating an aqueous dispersion solution containing the oxidized cellulose with a concentration of 0.1 mass% using a revolution/rotation stirrer under conditions of a revolving velocity of 2,000 rpm and a rotation velocity of 800 rpm for 10 minutes is preferably a value of 60% or more. The light transmittance of the nanocellulose aqueous dispersion solution is more preferably 70% or more, still more preferably 75% or more, and yet more preferably 80% or more. Here, the light transmittance is a value measured with a spectrophotometer at a wavelength of 660 nm.

Here, the reason why the oxidized cellulose in the present invention has excellent fibrillatability (particularly, ease-of-fibrillatability) and provides a high-quality slurry is not clear, but the following is generally considered. Fibrillation proceeds when hydrogen bonds between cellulose microfibrils break. In the oxidation treatment using the hypochlorous acid or salt thereof, the degree of polymerization of microfibrils decreases (that is, cellulose molecular chains become shorter) as the oxidation proceeds. It is thought that, in the present embodiment, when the number of hydrogen bonds to be broken by fibrillation in each microfibril according to the oxidation treatment is reduced, and additionally, the carboxy group content increases as the oxidation proceeds, the repulsive force between microfibrils is strengthened and fibrillatability of the oxidized cellulose is improved.

The oxidized cellulose in the present invention is obtained by oxidation with a hypochlorous acid or a salt thereof, and the oxidized cellulose obtained in this manner preferably has a structure in which at least two hydroxyl groups of the glucopyranose ring constituting cellulose are oxidized, and more specifically, has a structure in which hydroxyl groups at the 2nd position and the 3rd position of the glucopyranose ring are oxidized and carboxy groups are introduced. In addition, it is preferable that the hydroxyl group at the 6th position of the glucopyranose ring in the nanocellulose or oxidized cellulose not be oxidized and remain as a hydroxyl group. Here, the position of the carboxy group in the glucopyranose ring can be analyzed by comparing the solution NMR spectrum using a rayon oxide as a model molecule with the solid $^{13}$C-NMR spectrum of oxidized cellulose.

Rayon has the same chemical structure as cellulose, and its oxide (rayon oxide) is water-soluble. When rayon oxide is dissolved in heavy water and solution one-dimensional $^{13}$C-NMR measurement is performed, a carbon peak belonging to a carboxy group is observed at 165 to 185 ppm. In one aspect of oxidized cellulose or nanocellulose obtained by oxidizing raw material cellulose with the hypochlorous acid or salt thereof used in the present invention, two signals appear in this chemical shift range. In addition, according to solution two-dimensional NMR measurement, it can be determined that the carboxy group is introduced at the 2nd position and the 3rd position.

In solid $^{13}$C-NMR of oxidized cellulose or nanocellulose obtained by oxidizing raw material cellulose with the hypochlorous acid or salt thereof, when the amount of carboxy groups introduced is large, two signals appear at 165 to 185 ppm, and when the amount of carboxy groups introduced is small, a very broad signal may appear. As can be seen from the results of rayon oxide, signals of carboxy group carbon atoms introduced at the 2nd position and the 3rd position are close to each other, and separation of two signals is insufficient in solid $^{13}$C-NMR with low resolution. Therefore, when the amount of carboxy groups introduced is small, it is observed as a broad signal. That is, in the solid $^{13}$C-NMR spectrum, when the spread of peaks appearing at 165 to 185 ppm is evaluated, it can be confirmed that carboxy groups are introduced at the 2nd position and the 3rd position.

That is, after drawing a base line for peaks in the range of 165 ppm to 185 ppm in the solid $^{13}$C-NMR spectrum and obtaining a total area value, the ratio between two peak area values (large area value/small area value) obtained by vertically dividing the area value at the peak top is obtained, and if the ratio between the peak area values is 1.2 or more, it can be said that the peak is broad.

In addition, whether there is the broad peak can be determined by the ratio between the length L of the base line in a range of 165 ppm to 185 ppm and the length L' of the line perpendicular to the base line from the peak top. That is, if the ratio L'/L is 0.1 or more, it can be determined that there is a broad peak. The ratio L'/L may be 0.2 or more, 0.3 or more, 0.4 or more or 0.5 or more. The upper limit value of the ratio L'/L is not particularly limited, and may be generally 3.0 or less, 2.0 or less or 1.0 or less.

In addition, the structure of the glucopyranose ring can also be determined by analysis according to the method described in Sustainable Chem. Eng. 2020, 8, 48, 17800-17806.

Oxidized cellulose in the present invention is prepared without using N-oxyl compounds such as TEMPO. Therefore, oxidized cellulose and nanocellulose in the present invention are substantially free of N-oxyl compounds. Here, in the first invention, oxidized cellulose or nanocellulose being "substantially free of N-oxyl compounds" means that oxidized cellulose or nanocellulose contains no N-oxyl compounds or the content of N-oxyl compounds with respect to a total amount of oxidized cellulose or nanocellulose is 2.0 ppm by mass or less, and preferably 1.0 ppm by mass or less. In addition, when the content of N-oxyl compounds is preferably 2.0 ppm by mass or less, and more preferably 1.0 ppm by mass or less as an addition from the cellulose raw material, it means that it is "substantially free of N-oxyl compounds."

If it is substantially free of N-oxyl compounds, it is possible to prevent N-oxyl compounds, whose impacts on the environment and human body are a concern, from remaining in the oxidized cellulose or nanocellulose. The content of N-oxyl compounds can be measured by a known method. Examples of known methods include a method using a trace total nitrogen analysis device. Specifically, the nitrogen component derived from N-oxyl compounds in oxidized cellulose or nanocellulose can be measured using a trace total nitrogen analysis device (for example, device name: TN-2100H commercially available from Mitsubishi Chemical Analytech Co., Ltd.) as a nitrogen content.

[Nanocellulose]

The oxidized cellulose in the present invention may be refined into nanocellulose. One of the present invention provides a method of producing nanocellulose including a step of obtaining nanocellulose by fibrillating the oxidized cellulose obtained by the production method of the present invention. That is, the method of producing nanocellulose of the present invention includes a step of obtaining oxidized cellulose by oxidizing a cellulose raw material with a hypochlorous acid or a salt thereof and a step of obtaining nanocellulose by fibrillating the oxidized cellulose, and the oxidized cellulose contains an oxide of a cellulose raw material by the hypochlorous acid or salt thereof, is substantially free of N-oxyl compounds, and has a degree of polymerization of 600 or less, and the slurry viscosity or the initial viscosity of the reaction system is in a range of 1,000 Pa·s or less.

In addition, the oxidized cellulose in the present invention may be used by mixing it with other components. That is, a nanocellulose-containing composition containing nanocellulose and at least one other component can be obtained by performing mixing with other components without refining, and stirring oxidized cellulose by appropriate stirring or the like. In addition, the oxidized cellulose in the present invention can be made into nanocellulose by a user directly refining the oxidized cellulose when using it.

The nanocellulose in the present invention is derived from the oxidized cellulose obtained by the production method of the present invention and is obtained by fibrillating and refining the oxidized cellulose. Nanocellulose contains fine cellulose fibers.

The average fiber length of nanocellulose in the present invention is preferably 50 nm or more and 800 nm or less. If the average fiber length is 50 nm or more, the quality of nanocellulose tends to be uniform. In order to make the quality more uniform, the lower limit of the average fiber length is more preferably 100 nm or more, and still more preferably 150 nm or more. If the average fiber length is 800 nm or less, the proportion of coarse cellulose fibers is reduced, and the occurrence of precipitation of nanocellulose tends to be reduced. In order to further reduce the occurrence of precipitation, the upper limit of the average fiber length is more preferably 700 nm or less, and still more preferably 600 nm or less.

In order to further improve the quality of nanocellulose, the average fiber length is more preferably 50 nm or more and 700 nm or less, still more preferably 100 nm or more and 700 nm or less, and still more preferably 100 nm or more and 600 nm or less.

The average fiber width of nanocellulose in the present invention is preferably 1 nm or more and 100 nm or less. If the average fiber width is 1 nm or more, the quality of nanocellulose tends to be uniform. In order to make the quality more uniform, the lower limit of the average fiber width is more preferably 2 nm or more and still more preferably 3 nm or more. If the average fiber width is 100 nm or less, the proportion of coarse nanocellulose is reduced, and the occurrence of precipitation of nanocellulose tends to be reduced. In order to further reduce the occurrence of precipitation, the average fiber width is more preferably 50 nm or less, and still more preferably 30 nm or less.

In order to further improve the quality of nanocellulose, the average fiber width is more preferably 2 nm or more and 50 nm or less, and still more preferably 3 nm or more and 30 nm or less.

In the nanocellulose in the present invention, the aspect ratio (average fiber length/average fiber width), which is a ratio between the average fiber width and the average fiber length, is preferably 20 or more and 200 or less.

If the aspect ratio is 200 or less, the nanocellulose is uniformly dispersed and the quality tends to be improved. In view of this, the aspect ratio is more preferably 190 or less, and still more preferably 180 or less.

On the other hand, if the aspect ratio is too low, that is, if the shape of nanocellulose is a thick rod shape rather than an elongated fibrous shape, uneven distribution causes aggregation, and the quality of nanocellulose tends to decrease. Therefore, the aspect ratio is preferably 20 or more, more preferably 30 or more, and still more preferably 40 or more.

Here, the average fiber width and the average fiber length are values obtained by mixing nanocellulose and water so that the concentration of nanocellulose is about 1 to 10 ppm, naturally drying the sufficiently diluted cellulose aqueous dispersion on a mica substrate, observing the shape of nanocellulose using a scanning probe microscope, randomly selecting an arbitrary number of fibers from the obtained image, and calculating according to the cross section height of the shape image=fiber width and peripheral length÷2=fiber length. Image processing software can be used to calculate such an average fiber width and average fiber length. In this case, image processing conditions are arbitrary, but values calculated for the same image may differ depending on conditions. The range of difference in values depending on conditions is preferably within the range of ±100 nm for the average fiber length. The range of difference in values depending on conditions is preferably within the range of ±10 nm for the average fiber width. For a more detailed measurement method, the method described in examples to be described below will be used.

When various physical properties of nanocellulose in the present invention are measured, nanocellulose may be used as a measurement sample, a nanocellulose-containing composition may be used as a measurement sample, and nanocellulose after separating nanocellulose and other components (formulation) from the nanocellulose-containing composition may be used as a measurement sample.

As described above, in one aspect, the nanocellulose in the present invention can be characterized by an average fiber width, an average fiber length, or an aspect ratio, but in another aspect, a predetermined zeta potential and light transmittance may be used.

(Zeta potential)

In one embodiment of the present disclosure, the nanocellulose in the present invention preferably has a zeta potential of -30 mV or less. If the zeta potential is - 30 mV or less (that is, the absolute value is 30 mV or more), sufficient repulsion between microfibrils is obtained, and nanocellulose with a high surface charge density is likely to be produced. Thereby, the dispersion stability of nanocellulose is improved and the viscosity stability and handling properties when made into a slurry can be improved. In consideration of dispersion stability, the lower limit of the zeta potential is not particularly limited. However, if the zeta potential is -100 mV or more (that is, the absolute value is 100 mV or less), since oxidative cutting in the fiber direction tends to be minimized as oxidation proceeds, nanocellulose with a uniform size tends to be obtained.

For example, the zeta potential tends to be increased by setting one or more of the oxidation reaction time, reaction temperature and stirring conditions (for example, lengthening the reaction time) for the side on which oxidation is promoted (that is, the side on which the degree of oxidation increases). In addition, the zeta potential can be suitably controlled by oxidation with the hypochlorous acid or salt thereof.

In view of this, the zeta potential of nanocellulose in the present invention is more preferably -35 mV or less, still more preferably -40 mV or less, and yet more preferably -50 mV or less. In addition, the lower limit of the zeta potential is preferably -90 mV or more, more preferably -85 mV or more, still more preferably -80 mV or more, and yet more preferably -77 mV or more. The range of the zeta potential can be obtained by appropriately combining the above lower limits and upper limits.

The zeta potential is preferably -90 mV or more-35 mV or less, more preferably -85 mV or more-40 mV or less, and still more preferably -80 mV or more-50 mV or less. Here, in this specification, the zeta potential is a value measured under conditions of a pH of 8.0 and 20°C for a cellulose aqueous dispersion in which nanocellulose in the present invention and water are mixed and the nanocellulose concentration is 0.1 mass%.

The zeta potential can be measured in detail according to the following method.

Pure water is added to nanocellulose and dilution is performed so that the nanocellulose concentration is about 0.1%. 0.05 mol/L of an aqueous sodium hydroxide solution is added to the diluted nanocellulose aqueous dispersion, the pH is adjusted to about 8.0, and the zeta potential is measured at 20°C using, for example, a zeta potential meter (ELSZ-1000, commercially available from Otsuka Electronics Co., Ltd.).

(Light transmittance)

A nanocellulose dispersion obtained by dispersing the nanocellulose in the present invention in a dispersion medium can exhibit high light transmittance with little light scattering of cellulose fibers. Specifically, in one preferable embodiment, the nanocellulose in the present invention has a light transmittance of 95% or more in a mixed solution obtained by mixing with water and having a solid content concentration of 0.1 mass%. The light transmittance is more preferably 96% or more, still more preferably 97% or more, and yet more preferably 99% or more. Here, the light transmittance is a value measured with a spectrophotometer at a wavelength of 660 nm.

The light transmittance can be measured with a spectrophotometer (JASCO V-550), for example, by putting an aqueous nanocellulose dispersion into a quartz cell with a thickness of 10 mm.

The nanocellulose in the present invention is an assembly of single unit fibers. When carboxy groups are introduced into the nanocellulose in the present invention, at least one carboxylated nanocellulose (also referred to as carboxylated CNF) may be included, and the carboxylated nanocellulose is preferably a main component. Here, when the carboxylated CNF is a main component, it means that the proportion of carboxylated CNF with respect to a total amount of fine cellulose is more than 50 mass%, preferably more than 70 mass%, and more preferably more than 80 mass%. The upper limit of the proportion is 100 mass%, but it may be 98 mass% or 95 mass%.

A method of fibrillating oxidized cellulose is not particularly limited as long as it is an operation of dispersing nanocellulose. Here, the nanocellulose is a generic term for refined cellulose and includes cellulose nanofibers, cellulose nanocrystals and the like.

For fibrillation, for example, velocity fields and velocity fluctuations with arbitrary strength; collisions with inclusions and obstacles; ultrasonic waves; pressure loads; and the like can be used. A submerged dispersing machine can be suitably used for such a dispersing operation.

The submerged dispersing machine is not particularly limited, and for example, methods using a homomixer, a magnetic stirrer, a stirring bar, a stirrer with a stirring blade, a disper-type mixer, a homogenizer, external circulation stirring, a revolution/rotation stirrer, a vibration type stirrer, an ultrasonic dispersing machine and the like may be exemplified. In addition, as the submerged dispersing machine, in addition to the above devices, a rotary shear type stirrer, a colloid mill, a roll mill, a pressure type homogenizer, a container driven type mill, and a medium stirring mill may be exemplified. In addition, a kneader can be used as the submerged dispersing machine.

The rotary shear type stirrer is a device that performs dispersion by passing a material to be stirred through a gap between a rotor blade and an outer cylinder, and dispersion is performed with a shear flow in the gap and strong forward and backward velocity fluctuations.

The colloid mill is a device that performs dispersion with a shear flow in a gap between a rotating disk and a fixed disk. The roll mill performs dispersion with a shear force and a compression force using gaps between a plurality of rotating rollers.

The pressure type homogenizer is used as a dispersing machine that discharges a slurry or the like at a high pressure from pores, and is also called a pressure injection type dispersion instrument. The pressure type homogenizer is preferably a high pressure homogenizer. The high pressure homogenizer is, for example, a homogenizer having a function of discharging a slurry at a pressure of 10 MPa or more, preferably 100 MPa or more. Examples of high pressure homogenizers include counter-collision-type high pressure homogenizers such as a microfluidizer and a wet jet mill.

The container driven type mill is a device that performs dispersion by collision and friction of media such as balls in a container, and specific examples thereof include a rotary mill, a vibrating mill, and a planetary mill. The medium stirring mill is a device that performs dispersion with an impact force and a shear force of a medium using a medium such as a ball and a bead, and specific examples thereof include an attritor and a bead mill (sand mill).

The kneader is a device that performs an operation of wetting powder or the like with a liquid (hereinafter also referred to as kneading or blending), and specific examples thereof include a double arm kneading machine (a device that performs dispersion in two semi-cylindrical containers with twin-screw mixing blades); a Banbury mixer (closed system, a dispersing device under pressure); and extrusion kneading machines such as a screw extrusion machine, a co-kneader, and an extruder.

As the fibrillation method, methods using various mixing and stirring devices, for example, a screw type mixer, a paddle mixer, a disper-type mixer, a turbine type mixer, a homomixer under high speed rotation, a high pressure homogenizer, an ultra high pressure homogenizer, a double cylinder type homogenizer, an ultrasonic homogenizer, a water jet counter collision type dispersing machine, a beater, a disk type refiner, a conical type refiner, a double disk type refiner, a grinder, a single-screw or multi-screw kneader, a revolution/rotation stirrer, and a vibration type stirrer may be exemplified.

The devices used for fibrillation can be used alone or two or more types thereof can be used in combination.

For fibrillation of oxidized cellulose, for example, a method using an ultra high pressure homogenizer may be used because it enables nanocellulose with more advanced fibrillation to be produced. When fibrillation is performed using an ultra high pressure homogenizer, the pressure during a fibrillation treatment is preferably 100 MPa or more, more preferably 120 MPa or more, and still more preferably 150 MPa or more. The number of times the fibrillation treatment is performed is not particularly limited, and in order to allow fibrillation to sufficiently proceed, it is preferably 2 or more times, and more preferably 3 or more times.

Since oxidized cellulose has excellent ease-of-fibrillatability, it can be sufficiently fibrillated even when mild stirring using, for example, a revolution/rotation stirrer and a vibration type stirrer, is applied as the fibrillation method, and it is possible to obtain uniformized nanocellulose.

The revolution/rotation stirrer is a device that mixes materials in a container by rotating and revolving the container into which the materials are put. According to the revolution/rotation stirrer, since stirring is performed without using a stirring blade, milder stirring can be realized. The revolving velocity and the rotation velocity during stirring by the revolution/rotation stirrer can be appropriately set, and for example, the revolving velocity can be 400 to 3,000 rpm, and the rotation velocity can be 200 to 1,500 rpm. When the revolution/rotation stirrer is used, in order to realize mild stirring and secure uniformity of the quality, it is preferable to perform a fibrillation treatment under conditions of stirring at a revolving velocity of 1,200 to 2,500 rpm and a rotation velocity of 600 to 1,000 rpm for 3 to 15 minutes. The revolving velocity is more preferably 1,500 to 2,300 rpm, and the rotation velocity is more preferably 700 to 950 rpm. When the present oxidized cellulose fibers are fibrillated using the revolution/rotation stirrer, the concentration of the oxidized cellulose aqueous dispersion as a material is, for example, 0.01 to 1.0 mass%, and preferably 0.1 to 0.5 mass%.

Examples of vibration type stirrers include a vortex mixer (touch mixer). In the vortex mixer, stirring is performed by forming a vortex in the liquid material in the container. According to the vibration type stirrer such as a vortex mixer, since stirring is performed without using a stirring blade, milder stirring can be realized. In addition, the vibration type stirrer such as a vortex mixer is excellent in terms of production instruments and production cost because since mild stirring can be realized with a simple instrument. The rotational speed of the vortex mixer is, for example, 600 to 3,000 rpm, and the fibrillation treatment is preferably performed under conditions of stirring for 3 to 15 minutes. When the present oxidized cellulose fibers are fibrillated using a vortex mixer, the concentration of the oxidized cellulose aqueous dispersion as a material is, for example, 0.01 to 1.0 mass%, and preferably 0.1 to 0.5 mass%.

The fibrillation treatment is preferably performed when the oxidized cellulose is mixed with a dispersion medium. The dispersion medium is not particularly limited and can be appropriately selected depending to the purpose. Specific examples of dispersion media include water, alcohols, ethers, ketones, N,N-dimethylformamide, N,N-dimethylacetamide, and dimethyl sulfoxide. As the solvent, these examples may be used alone or two or more thereof may be used in combination.

Among the above dispersion media, examples of alcohols include methanol, ethanol, isopropanol, isobutanol, sec-butyl alcohol, tert-butyl alcohol, methyl cellosolve, ethylene glycol and glycerin. Examples of ethers include ethylene glycol dimethyl ether, 1,4-dioxane and tetrahydrofuran. Examples of ketones include acetone and methyl ethyl ketone.

When an organic solvent is used as a dispersion medium during the fibrillation treatment, oxidized cellulose and nanocellulose obtained by fibrillating it are easily isolated. In addition, since nanocellulose dispersed in an organic solvent can be obtained, it is easy to mix it with a resin that dissolves in an organic solvent, a resin raw material monomer or the like. A nanocellulose dispersion solution obtained by dispersing the nanocellulose obtained by fibrillation in a dispersion medium such as water and/or an organic solvent can be used for mixing with various components such as a resin, rubber, and solid particles.

The oxidized cellulose and nanocellulose obtained by the production method of the present invention can be applied for various applications. Specifically, for example, they may be used as various materials (for example, resins, fibers, rubber, etc.) and may be used for various applications (for example, food, cosmetics, medical products, paints, inks, etc.). In addition, the nanocellulose-containing composition can be formed into a film and used as various sheets or films. The fields in which the nanocellulose-containing composition is applied are not particularly limited, and it can be used in production of products in various fields, for example, automobile members, machine parts, electrical appliances, electronic device, cosmetics, medical products, building materials, daily necessities, stationery and the like.

<<Embodiment for performing second invention>>

<Method of producing oxidized cellulose>

A method of producing oxidized cellulose of the present invention includes a step of obtaining oxidized cellulose by solid-liquid separation of an oxide dispersion containing a cellulose-based oxide and a dispersion medium (hereinafter referred to as a "separating step"). In addition, the pH of the oxide dispersion is 4.0 or less.

In addition, the oxide dispersion is substantially free of N-oxyl compounds or the method further includes a step of obtaining the cellulose-based oxide by oxidizing a cellulose raw material with a predetermined amount range of the hypochlorous acid or salt thereof.

Here, in the second invention, the meaning of "substantially free of N-oxyl compounds" is the same as described in the above <<Embodiment for performing first invention>> (replacing "oxidized cellulose or nanocellulose" with "oxide dispersion").

According to the method of producing oxidized cellulose of the present invention, it is possible to obtain oxidized cellulose at a high yield. The reason for this is inferred as follows (however, the reason is not limited to this). In the conventional production method, if solid-liquid separation is performed when a cellulose-based oxide obtained by oxidizing a cellulose raw material with a hypochlorous acid or a salt thereof is dispersed in a dispersion medium, the cellulose-based oxide is refined in the dispersion medium, a part thereof is transferred from a solid phase to a liquid phase, and thus the yield of the obtained oxidized cellulose is reduced. Particularly, when solid-liquid separation is performed by filtration, clogging may occur in the filter cloth, which makes solid-liquid separation itself difficult. On the other hand, according to the method of producing oxidized cellulose of the present invention, mainly because the pH of the oxide dispersion is 4.0 or less, it is possible to prevent the cellulose-based oxide from being refined in the dispersion medium and improve the yield of oxidized cellulose collected by solid-liquid separation. Particularly, when solid-liquid separation is performed by filtration, clogging does not occur in the filter cloth, the operability of the solid-liquid separation is improved, and the oxidized cellulose is then easily washed on the filter cloth.

Hereinafter, for each step of an example shown in the flowchart in Fig. 1, the production method of the present invention will be described in detail, but the present invention is not limited thereto and various modifications can be made without departing from the spirit and scope of the present invention.

[Oxidizing step]

The production method of the present invention may include a step of obtaining a cellulose-based oxide by oxidizing a cellulose raw material (hereinafter referred to as an "oxidizing step") in order to prepare a cellulose-based oxide used in the separating step. In the oxidizing step, an oxidant can be used to oxidize the cellulose raw material, and particularly, it is preferable to use a hypochlorous acid or a salt thereof.

When oxidizing is performed using the hypochlorous acid or salt thereof, it is possible to obtain a cellulose-based oxide without using N-oxyl compounds such as 2,2,6,6-tetramethyl-1-piperidine-N-oxy radical (TEMPO) as an oxidant.

Specific examples of the hypochlorous acid or salt thereof are the same as described in the above <<Embodiment for performing first invention>>.

Specific examples of cellulose raw materials are the same as described in the above <<Embodiment for performing first invention>>.

In the relation to the second invention, the cellulose-based oxide is a material mainly composed of cellulose containing carboxy groups. In the separating step to be described below, it is preferable to use the cellulose-based oxide obtained in the oxidizing step, but there is no particular limitation.

In the relation to the second invention, the oxide dispersion is one containing a cellulose-based oxide and a dispersion medium and having a pH of 4.0 or less.

In the relation to the second invention, the oxidized cellulose is a component that can be incorporated into a solid phase in a dispersion among materials mainly composed of cellulose containing carboxy groups. For example, among cellulose polymerization components, it is a component that does not dissolve in a dispersion medium in a dispersion and is extracted as a solid phase because it has a predetermined degree of polymerization or more, and less than a predetermined value of water solubility.

Here, in this specification, the pH can be measured with a pH meter with a pH electrode. In addition, the pH range can be controlled using a pH controller with a pH electrode.

Examples of methods of obtaining a cellulose-based oxide by oxidizing a cellulose raw material include a method of mixing a cellulose raw material and a reaction solution containing a hypochlorous acid or a salt thereof. The solvent contained in the reaction solution is preferably water because it is easy to handle and side reactions are unlikely to occur.

In the oxidizing step, the hypochlorous acid or salt thereof is not particularly limited, and the mass proportion with respect to the cellulose raw material is preferably 0.2 or more, more preferably 0.5 or more, and still more preferably 1.0 or more. Within this range, the carboxy group content in the obtained cellulose-based oxide and oxidized cellulose can be sufficiently increased, and sufficient refining during fibrillation to be described below tends to proceed. The upper limit of the mass proportion is not particularly limited, and preferably 20 or less, more preferably 10 or less, and still more preferably 5.0 or less. The range of the mass proportion may be determined by appropriately combining the above upper limit values and lower limit values, and may be, for example, 0.2 or more and 20 or less, 0.5 or more and 10 or less, or 1.0 or more and 5.0 or less.

Oxidized cellulose is obtained, for example, by oxidizing a cellulose raw material under the above mass proportion condition and subjecting the separating step to be described below, and can also be produced by appropriately controlling reaction conditions such as the mass proportion, the pH during the reaction, and the reaction temperature. The structure of the oxidized cellulose obtained in this manner (oxidation state of hydroxyl groups) is the same as described in the above

<<Embodiment for performing first invention>>.

In the oxidizing step, adjustment of pH and the range of pH are arbitrary, and a preferable pH value and a pH adjusting method are the same as described in the above <<Embodiment for performing first invention>>.

[Treatment step]

The production method of the present invention may further include a step of treating the hypochlorous acid or a salt thereof (hereinafter referred to as a "treating step") used in the oxidizing step. When the method includes the treating step, the reaction of oxidizing the cellulose raw material can be stopped. Specific examples of treating steps are the same as described in the above <<Embodiment for performing first invention>>.

[Protonating step]

The production method of the present invention may further include a step of adding an acid to prepare an oxide dispersion having a pH of 4.0 or less (hereinafter referred to as a "protonating step") in order to prepare an oxide dispersion used in the separating step. Here, the cellulose-based oxide contained in the oxide dispersion contains carboxy groups, and the protonating step is a step of converting at least some of carboxy groups from the salt form (-COO- X+: X+ indicates a cation such as sodium) to the proton form (-COO-H+).
The acid used in the protonating step is not particularly limited as long as it can be used to prepare an oxide dispersion having a pH of 4.0 or less, and examples thereof include inorganic acids and organic acids. Among these, in consideration of ease of handling, an inorganic acid, and particularly, hydrochloric acid, is preferable. In addition, in the protonating step, a cation ion exchange resin may be used.
As the cation ion exchange resin, both a strongly acidic ion exchange resin and a weakly acidic ion exchange resin can be used as long as the counterion is H+, and among these, a strongly acidic ion exchange resin is preferable. Examples of strongly acidic ion exchange resins and weakly acidic ion exchange resins include those obtained by introducing a sulfonic acid group or a carboxy group into a styrene resin or an acrylic resin. The form of the cation ion exchange resin is not particularly limited, and various forms such as a fine granular form (a granular form), a film form, and a fiber form can be used. Among these, a granular form is preferable because a carboxylated cellulose nanofiber salt is efficiently desalted and separation after the desalting treatment becomes easier. A commercial product can be used as such a cation ion exchange resin. Examples of commercial products include Amberjet 1020, Amberjet 1024, Amberjet 1060, and Amberjet 1220 (all are commercially available from Organo Corporation), Amberlite IR-200C and Amberlite IR-120B (all are commercially available from Tokyo Organic Chemical Co., Ltd.), Lewatit SP112 and Lewatit S100 (all are commercially available from Bayer AG), GELCK08P (commercially available from Mitsubishi Chemical Corporation), and Dowex50W-X8 (commercially available from The Dow Chemical Company).
After protonating is performed using a cation ion exchange resin, the cation ion exchange resin may be removed by filtration through a metal mesh or the like.
The dispersion medium used in the protonating step is not particularly limited, and it is preferable to use the solvent contained in the acid without change. In addition, depending on the purpose, a dispersion medium not contained in the acid can be used together appropriately or a solvent contained in the acid can be replaced with another dispersion medium. Specific examples of dispersion media include those described as the dispersion solution during the fibrillation treatment in the above <<Embodiment for performing first invention>>.
The pH of the oxide dispersion is preferably 4.0 or less, more preferably 3.0 or less, and still more preferably 2.5 or less. In this case, the lower limit of the pH of the oxide dispersion is not particularly limited, and is generally 1.0 or more, preferably 1.5 or more, and more preferably 2.0 or more. The range of the pH of the oxide dispersion may be obtained by appropriately combining the above upper limit values and lower limit values, and may be, for example, 1.0 or more and 4.0 or less, 1.5 or more and 3.0 or less, or 2.0 or more and 2.5 or less.
When the method includes a protonating step, the protonating step may be performed before the separating step and preferably after the oxidizing step and may be performed at the same time as a part of the oxidizing step.
The cellulose-based oxide may be used in the separating step after it is additionally purified as necessary before, after or during the protonating step. In addition, the solution containing the cellulose-based oxide obtained in the oxidizing step may be subjected to the separating step without change.

[Separating step]

The method of producing oxidized cellulose of the present invention includes a step of obtaining oxidized cellulose by solid-liquid separation of an oxide dispersion containing a cellulose-based oxide and a dispersion medium (separating step). In addition, the pH of the oxide dispersion is 4.0 or less.

The method of performing solid-liquid separation of an oxide dispersion is not particularly limited, and examples thereof include a method of obtaining oxidized cellulose contained in a solid phase by removing a liquid phase by known isolation treatments such as centrifugation and filtration. Among these, solid-liquid separation by filtering the oxide dispersion is preferable in consideration of operability.

The pH of the oxide dispersion in the separating step is the same as the pH of the oxide dispersion prepared in the protonating step, and a specific value is as described above.

The dispersion medium used in the separating step is the same as the dispersion medium used in the protonating step, and it is preferable to use the dispersion medium used there without change. Specific examples of dispersion media are the same as the dispersion media used in the protonating step.

[Washing step]

The method of producing oxidized cellulose of the present invention may further include a step of washing an oxide dispersion or oxidized cellulose with an acidic washing solution (hereinafter referred to as a "washing step").

As the acidic washing solution used in the washing step, a solution containing the acid used in the protonating step and a dispersion medium can be used, but is not particularly limited.

The pH of the acidic washing solution is the same as the pH of the above oxide dispersion, and a specific value is as described above.

When the method includes a washing step, the washing step may be performed before, after or at the same time as the separating step, and as will be described below in examples, an operation of repeating the separating step and the washing step may be performed.

[Salifying step]

The production method of the present invention may further include a step of adding a base to adjust the pH of the oxidized cellulose dispersion containing the oxidized cellulose and dispersion medium obtained in the separating step to more than 4.0 (hereinafter referred to as a "salifying step," and the salifying step is also referred to as a "neutralizing step"). Here, the oxidized cellulose contained in the oxidized cellulose dispersion contains carboxy groups, and the salifying step is a step of converting at least some of carboxy groups from the proton form (-COO-H+) to the salt form (-COO- X+: X+ indicates a cation such as sodium or lithium).

The base used in the salifying step is not particularly limited as long as the pH of the oxidized cellulose dispersion can be adjusted to more than 4.0, and examples thereof include inorganic bases and organic bases. Among these, in consideration of ease of handling, an inorganic base, particularly, a sodium hydroxide, is preferable.

In addition, amines can be used as the base. The amines may be primary amines, secondary amines, tertiary amines or quaternary amines.

The pH of the oxidized cellulose dispersion is preferably 5.0 or more, more preferably 6.0 or more, and still more preferably 7.0 or more. The upper limit value of the pH is not particularly limited, and is preferably 14.5 or less, more preferably 14.0 or less, still more preferably 12.0 or less, yet more preferably 10.0 or less, yet more preferably 9.0 or less, and particularly preferably 8.0 or less. The range of the pH may be obtained by appropriately combining the above upper limit values and lower limit values, and may be, for example, 5.0 or more and 14.5 or less, 5.0 or more and 14.0 or less, 6.0 or more and 12.0 or less, 6.0 or more and 10.0 or less, 7.0 or more and 9.0 or less, or 7.0 or more and 8.0 or less.

The dispersion medium used in the salifying step is not particularly limited, and it is preferable to use the solvent contained in the base without change. In addition, depending on the purpose, a dispersion medium not contained in the base can be used together appropriately or a solvent contained in the base can be replaced with another dispersion medium. Specific examples of dispersion media are the same as the dispersion media used in the protonating step. However, water and/or an organic solvent is preferable because it facilitates isolation of nanocellulose in the fibrillation step to be described below.

When the method includes a salifying step, the salifying step may be performed after the separating step or may be performed before, after or at the same time as the washing step.

<Oxidized cellulose>

The oxidized cellulose of the present invention is oxidized cellulose obtained by the production method of the present invention. Specifically, the oxidized cellulose is a component derived from the oxide dispersion used in the separating step and extracted as a solid phase according to a solid-liquid separation operation.

The oxidized cellulose is preferably in the form of a slurry. The slurry referred to here is a suspension containing oxidized cellulose. The slurry may contain the dispersion medium used in the separating step. In addition, a dispersion medium may be appropriately added to form a slurry. Since the oxidized cellulose is a slurry, it is easy to handle and tends to be easily refined.

The oxidized cellulose includes fibrous cellulose obtained by oxidizing a cellulose raw material with a hypochlorous acid or a salt thereof. The oxidized cellulose in the present invention is also called an oxidized cellulose fiber. That is, the oxidized cellulose in the present invention includes an oxide of the cellulose raw material by the hypochlorous acid or salt thereof.

A preferable degree of polymerization of the oxidized cellulose, its adjustment method and its measurement method are the same as described in the above

<<Embodiment for performing first invention>>.

A preferable light transmittance of the nanocellulose aqueous dispersion solution obtained by fibrillating an aqueous dispersion solution containing oxidized cellulose with a concentration of 0.1 mass% using a revolution/rotation stirrer under conditions of a revolving velocity of 2,000 rpm, and a rotation velocity of 800 rpm for 10 minutes is the same as described in the above <<Embodiment for performing first invention>>.

In addition, the oxidized cellulose of the present invention may be used by mixing it with other components. That is, a nanocellulose-containing composition containing nanocellulose and at least one other component can be obtained by performing mixing with other components without refining, and stirring oxidized cellulose by appropriate stirring or the like. In addition, the oxidized cellulose in the present invention can be made into nanocellulose by a user directly refining the oxidized cellulose when using it.

<Method of producing nanocellulose>

The method of producing nanocellulose of the present invention includes a step of obtaining nanocellulose by fibrillating the oxidized cellulose obtained by the method of producing oxidized cellulose of the present invention.

The fibrillation method is not particularly limited as long as it is a method of refining oxidized cellulose, and is preferably performed when oxidized cellulose is mixed with a dispersion medium. A specific fibrillation method is the same as described in the above <<Embodiment for performing first invention>>.

The dispersion medium used in the fibrillation step is the same as the dispersion medium used in the salifying step, and it is preferable to use the dispersion medium used there without change. Specific examples of dispersion media are the same as the dispersion media used in the protonating step.

However, water and/or an organic solvent is preferable because it facilitates isolation of nanocellulose. In addition, since nanocellulose dispersed in an organic solvent can be obtained, it is easy to mix it with a resin that dissolves in an organic solvent, a resin raw material monomer or the like. A nanocellulose dispersion solution obtained by dispersing the nanocellulose obtained by fibrillation in a dispersion medium such as water and/or an organic solvent can be used for mixing with various components such as a resin, rubber, and solid particles.

<Nanocellulose>

The nanocellulose of the present invention is nanocellulose obtained by the method of producing nanocellulose of the present invention, and is derived from the oxidized cellulose of the present invention, and is obtained by fibrillating and refining the oxidized cellulose. In this specification, the nanocellulose is a generic term for refined cellulose and includes fine cellulose fibers, cellulose nanocrystals and the like. Fine cellulose fibers are referred to as cellulose nanofibers (also called CNF).

The average fiber length and the average fiber width of nanocellulose, the methods of measuring them, and the aspect ratio (average fiber length/average fiber width) are the same as described in the above <<Embodiment for performing first invention>>.

Nanocellulose preferably exhibits a light transmittance value of 60% or more in an aqueous dispersion solution. The light transmittance of the nanocellulose aqueous dispersion solution is more preferably 70% or more, still more preferably 75% or more, and yet more preferably 80% or more. A specific method of measuring light transmittance will be described in examples to be described below.

Applications of the oxidized cellulose and nanocellulose obtained by the production method of the present invention are the same as described in the above

<<Embodiment for performing first invention>>.

<<Combination of first invention and second invention>>

The first invention and the second invention may be combined to provide a method including a step of obtaining oxidized cellulose by oxidizing a cellulose raw material and a step of post-treating the oxidized cellulose. As an example, the following method may be exemplified, but the present invention is not limited thereto, and the specific embodiment of the first invention and the specific embodiment of the second invention can be appropriately combined.

[Example of combination]

A method of producing oxidized cellulose, which is substantially free of N-oxyl compounds and has a degree of polymerization of 600 or less, includes a step of obtaining first oxidized cellulose by oxidizing a cellulose raw material with a hypochlorous acid or a salt thereof (here, the viscosity of the slurry of the cellulose raw material having the same concentration as when the oxidation is performed, which is measured using a viscometer including an SPP rotor, is in a range of 1,000 Pa·s or less under measurement conditions of a rotational speed of 100 rpm, and 30°C or 40°C) [Part of first invention], and
a step of obtaining second oxidized cellulose by solid-liquid separation of an oxide dispersion containing the first oxidized cellulose and a dispersion medium (here, the pH of the oxide dispersion is 4.0 or less) [Part of second invention].
Here, "first oxidized cellulose" and "second oxidized cellulose" in the above [Part of second invention] correspond to "cellulose-based oxide" and "oxidized cellulose" in the above <<Embodiment for performing second invention>>.

Examples

Hereinafter, the present invention will be described in detail with reference to examples, but the present invention is limited to these examples. Here, in the following, unless otherwise specified, "parts" means "parts by mass," and "%" means "mass%."
Various physical properties were measured as follows.

[Measurement of viscosity average degree of polymerization of oxidized cellulose]

Oxidized cellulose was added to an aqueous sodium borohydride solution whose pH was adjusted to 10 and a reduction treatment was performed at 25°C for 5 hours. The amount of sodium borohydride was 0.1 g per 1 g of oxidized cellulose fibers. After the reduction treatment, solid-liquid separation was performed by suction filtration, washing with water was performed, and the obtained oxidized cellulose fibers were freeze-dried. 0.04 g of the dried oxidized cellulose fibers were added to 10 ml of pure water, and the mixture was stirred for 2 minutes, and 10 ml of a 1 mol/L copper ethylenediamine solution was then added and dissolved. Then, a flow-down time of a blank solution and a flow-down time of a cellulose solution were measured at 25°C using a capillary type viscometer. From the flow-down time ($t0$) of the blank solution, the flow-down time ($t$) of the cellulose solution, and the concentration ($c$[g/ml]) of the oxidized cellulose fiber, the relative viscosity ($\eta r$), the specific viscosity ($\eta sp$), and the intrinsic viscosity ($[\eta]$) were obtained sequentially according to the following formula, and the degree of polymerization (DP) of the oxidized cellulose fibers was calculated from the viscosity formula.

$$\eta r = \eta/\eta 0 = t/t0$$

$$\eta sp = \eta r - 1$$

$$[\eta] = \eta sp/(100 \times c(1+0.28\ \eta sp))$$

$$DP = 175 \times [\eta]$$

[Measurement of average fiber width and average fiber length]

Pure water was added to an aqueous nanocellulose dispersion, and the concentration of oxidized CNFs in the oxidized CNF aqueous dispersion was adjusted to 5 ppm. The CNF aqueous dispersion after the concentration was adjusted was naturally dried on a mica substrate and the shape of oxidized CNFs was observed in an AC mode using a scanning probe microscope ("MFP-3D infinity" commercially available from Oxford Asylum).
For the average fiber length, the obtained image was binarized and analyzed using image processing software "Imaged." For 100 or more fibers, the average fiber length was determined according to fiber length="peripheral length"-2.
For the average fiber width, using software bundled in "MFP-3D infinity," for 50 or more fibers, the number average fiber width [nm] was determined according to cross section height of shape image=fiber width.

[Measurement of viscosity of reaction system]

First, a slurry composed of only a cellulose raw material and water was prepared. Specifically, the powdered pulp (VP-1) used in examples/comparative examples was used to prepare slurries having 7 mass%, 15 mass%, and 20 mass%, which were the same concentrations of the cellulose raw material in the reaction system of examples/comparative examples. Using these slurries, the viscosity was measured at 30°C or 40°C.
The viscosity of the slurry was measured using a viscometer with a scrolled parallel plate (SPP) rotor (RE-85U, commercially available from Toki Sangyo Co., Ltd.) while stirring at a rotational speed of 100 rpm (200 s$^{-1}$ in terms of shear rate). Here, as the SPP rotor, a spiral-grooved parallel disk rotor system with a rotor diameter of $\varphi$19.4 (commercially available from Toki Sangyo Co., Ltd.) was used.
Here, the viscosity was measured after the cellulose raw material and water were completely mixed.
The viscosities of the 7 mass%, 15 mass%, and 20 mass% slurries at 30°C were 1.00 Pa·s or less, 1.91 Pa·s, and 9.11 Pa·s, respectively.
The viscosities of the 7 mass%, 15 mass%, and 20 mass% slurries at 40°C were 1.00 Pa·s or less, 2.32 Pa·s, and 11.91 Pa·s, respectively.

[Light transmittance of nanocellulose]

Pure water was added to nanocellulose to prepare an aqueous dispersion with a CNF concentration of 0.1 mass%. This aqueous dispersion was put into a quartz cell with a thickness of 10 mm, and the value of the transmittance at a wavelength of 660 nm measured with a spectrophotometer (JASCO V-550) was used as the light transmittance.

[Degree of crystallization of cellulose raw material]

The degree of crystallization was calculated from the peak of the 4th carbon (hereinafter referred to as C4) of cellulose by performing solid $^{13}$C-NMR measurement on the freeze-dried cellulose raw material. The C4 peak appeared in a range of about 80 to 95 ppm in the form in which peaks of a crystal part (high ppm side, about 85 to 95 ppm) and a non-crystal part (low ppm side) overlapped, and was divided into respective peak areas (crystal part: SC, non-crystal part: SA) by a vertical dividing method. The degree of crystallization is obtained by the following formula.

$$\text{degree of crystallization}=SC/(SC+SA)\times100$$

solid $^{13}$C-NMR measurement was performed under the following conditions.

device: JNM-ECA, JEOL
frequency: 15 kHz
measurement method: CP/MAS method
waiting time: 5 seconds
cumulative number of measurements: 10,000

<Examples and comparative examples of first invention>

[Example 1A]

780 g of sodium hypochlorite pentahydrate crystals with an available chlorine concentration of 42 mass% was put into a 2 L baffled jacketed glass container, and pure water was added and stirred to adjust the available chlorine concentration to 21 mass%. 35 mass% hydrochloric acid was added thereto and stirred to obtain an aqueous sodium hypochlorite solution with a pH of 11.

The aqueous sodium hypochlorite solution was stirred at 300 rpm with a stirrer (three-one motor, BL600 commercially available from Shinto Scientific Co., Ltd.) using three swept blades, 30°C water was circulated through the jacket, heating was performed to 30°C, and 118 g of powdered pulp (VP-1, degree of crystallization: 40%, commercially available from TDI Corporation) was then added as the cellulose raw material.

After the cellulose raw material was supplied, while the temperature was maintained at 30°C, the pH during the reaction was adjusted to 11 while adding 25 mass% sodium hydroxide, and the mixture was stirred with a stirrer for 2 hours under the same conditions. In this case, no problem was observed in the stirring of the reaction system. Here, since the initial concentration of the cellulose raw material in the slurry during the reaction was 7 mass%, the viscosity of the reaction system was considered to be 1.00 Pa·s or less based on the viscosity measured according to the above [Measurement of viscosity of reaction system].

After the reaction was completed, oxidized cellulose was collected by repeating centrifugation (1000G, 10 minutes), decantation, and addition of pure water in an amount corresponding to the removed liquid. Water was added to adjust the oxidized cellulose concentration to 1%, fibrillation was performed with a homomixer under dispersion treatment conditions of 10,000 rpm and 10 minutes to obtain an aqueous nanocellulose dispersion. As a result of analyzing the aqueous dispersion, it was found to be nanocellulose with an average fiber length of 165 nm and an average fiber width of 3.2 nm.

In addition, the degree of polymerization of the oxidized cellulose was 96.

Here, the available chlorine concentration in the aqueous sodium hypochlorite solution was measured by the following method.

(Measurement of available chlorine concentration in aqueous sodium hypochlorite solution)

Sodium hypochlorite pentahydrate crystals were added to pure water, 0.582 g of an aqueous solution was accurately weighed, 50 ml of pure water was added, 2 g of potassium iodide and 10 ml of acetic acid were added, sealing was immediately performed, and the sample was left in a dark place for 15 minutes. After being left for 15 minutes, the released iodine was titrated with a 0.1 mol/L sodium thiosulfate solution (indicator starch test solution), and as a result, the titration amount was 34.55 ml. A blank test was separately performed to perform correction and since 1 ml of a 0.1 mol/L sodium thiosulfate solution corresponded to 3.545 mgCl, the available chlorine concentration in the aqueous sodium hypochlorite solution was 21 mass%.

Samples obtained by freeze-drying the oxidized cellulose obtained in respective production examples and then leaving them at 23°C, 50% RH for 24 hours or longer were subjected to solid $^{13}$C-NMR measurement, and as a result, it was confirmed that all had a structure in which hydroxyl groups at the 2$^{nd}$ position and the 3$^{rd}$ position of the glucopyranose ring were oxidized and carboxy groups were introduced. Solid $^{13}$C-NMR measurement conditions are as follows.

(1) sample tube: zirconia tube (4 mm diameter)

(2) magnetic field strength: 9.4T (1H resonance frequency: 400 MHz)

(3) MAS rotational speed: 15 kHz

(4) pulse sequence: CPMAS method

(5) contact time: 3 ms

(6) waiting time: 5 seconds

(7) cumulative number of measurements: 10,000 to 15,000

(8) measurement device: JNM ECA-400 (commercially available from JEOL Ltd.)

[Example 2A]

The conditions were the same as in Example 1A except that the amount of powdered pulp was changed to 275 g. Here, since the initial concentration of the cellulose raw material in the slurry during the reaction was 15 mass%, the viscosity of the reaction system was considered to be 1.91 Pa·s based on the viscosity measured according to the above [Measurement of viscosity of reaction system]. No problem was observed in the stirring of the reaction system, and as a result of analyzing the aqueous dispersion of nanocellulose obtained by fibrillation under the same conditions, it was found to be nanocellulose with an average fiber length of 168 nm and an average fiber width of 3.4 nm.

In addition, the degree of polymerization of the oxidized cellulose was 105.

[Example 3A]

780 g of a sodium hypochlorite pentahydrate crystal with an available chlorine concentration of 42 mass% was put into a 4 L baffled jacketed glass container, and pure water was added and stirred to adjust the available chlorine concentration to 13 mass%. 35 mass% hydrochloric acid was added thereto and stirred to obtain an aqueous sodium hypochlorite solution having a pH of 10.

The aqueous sodium hypochlorite solution was stirred at 300 rpm with a stirrer (three-one motor, BL600 commercially available from Shinto Scientific Co., Ltd.) using three swept blades, 40°C water was circulated through the jacket, heating was performed to 40°C, and 190 g of powdered pulp (VP-1, degree of crystallization: 40%, commercially available from TDI Corporation) was then added as the cellulose raw material.

After the cellulose raw material was supplied, while the temperature was maintained at 40°C, the pH during the reaction was adjusted to 10 while adding 25 mass% sodium hydroxide, and the mixture was stirred with a stirrer for 4 hours under the same conditions. In this case, no problem was observed in the stirring of the reaction system. Here, since the initial concentration of the cellulose raw material in the slurry during the reaction was 7 mass%, the viscosity of the reaction system was considered to be 1.00 Pa·s or less based on the viscosity measured according to the above [Measurement of viscosity of reaction system].

After the reaction was completed, oxidized cellulose was collected by repeating centrifugation (1000G, 10 minutes), decantation, and addition of pure water in an amount corresponding to the removed liquid. Water was added to adjust the oxidized cellulose concentration to 1%, fibrillation was performed with a homomixer under dispersion treatment conditions of 10,000 rpm and 10 minutes to obtain an aqueous nanocellulose dispersion. As a result of analyzing the aqueous dispersion, it was found to be nanocellulose with an average fiber length of 174 nm and an average fiber width of 4.2 nm.

In addition, the degree of polymerization of the oxidized cellulose was 101.

[Example 4A]

The conditions were the same as in Example 3A except that the amount of powdered pulp was changed to 445 g. Here, since the initial concentration of the cellulose raw material in the slurry during the reaction was 15 mass%, the viscosity of the reaction system was considered to be 2.32 Pa·s based on the viscosity measured according to the above [Measurement of viscosity of reaction system].

No problem was observed in the stirring of the reaction system, and as a result of analyzing the aqueous dispersion of nanocellulose obtained by fibrillation under the same conditions as in Example 3A, it was found to be nanocellulose with an average fiber length of 181 nm and an average fiber width of 4.5 nm.

In addition, the degree of polymerization of the oxidized cellulose was 114.

[Example 5A]

The procedure was performed in the same manner as in Example 1A except that the concentration of the cellulose raw material in the reaction system was set to 20 mass% with respect to a total amount of the reaction system. The viscosity of the reaction system was considered to be 9.11 Pa·s based on the viscosity measured according to the above [Measurement of viscosity of reaction system]. Since the viscosity gradually decreased, the progress of the oxidation reaction was confirmed, but it was difficult to stir the reaction system.

[Example 6A]

The procedure was performed in the same manner as in Example 3A except that the concentration of the cellulose raw material in the reaction system was set to 20 mass% with respect to a total amount of the reaction system. The viscosity of the reaction system was considered to be 11.91 Pa·s based on the viscosity measured according to the above [Measurement of viscosity of reaction system]. Since the viscosity gradually decreased, the progress of the oxidation reaction was confirmed, but it was difficult to stir the reaction system.

[Comparative Example 1A]

If the viscosity was more than 1,000 Pa·s, the mixture could not be stirred even with a kneader, and the oxidation reaction could not be performed.

<Examples and comparative examples of second invention>

[Example 1B]

(Oxidizing step)

350 g of a sodium hypochlorite pentahydrate crystal with an available chlorine concentration of 42 mass% was put into a beaker, and pure water was added and stirred to adjust the available chlorine concentration to 21 mass%. 35 mass% hydrochloric acid was added thereto and stirred to obtain an aqueous sodium hypochlorite solution having a pH of 11.

The aqueous sodium hypochlorite solution was stirred at 200 rpm with a stirrer (three-one motor, BL600, commercially available from Shinto Scientific Co., Ltd.) using propeller type stirring blades, heating was performed to 30°C in a constant temperature water bath, and 50 g of powdered pulp (VP-1, degree of crystallization: 40%, commercially available from TDI Corporation) was then added as the cellulose raw material. After the cellulose raw material was supplied, while maintaining conditions in which the temperature was maintained at 30°C in the constant temperature water bath, and the pH of the reaction system was adjusted to 11 while adding 48 mass% sodium hydroxide, the mixture was stirred with a stirrer for 2 hours. Then, pure water was added to perform 2-fold dilution, sodium hydroxide was added to adjust the pH to 13, the oxidation reaction was slowed down, and a cellulose-based oxide dispersed in water was obtained.

(Treatment step)

An aqueous sodium sulfite solution was added to the obtained cellulose-based oxide dispersed in water to reduce the residual excess sodium hypochlorite content.

(Protonating step)

Then, hydrochloric acid was added to convert carboxy groups of the cellulose-based oxide from the salt form (-COO-Na+) to the proton form (-COO-H+) to obtain an aqueous dispersion having a pH of 2.5.

Here, the pH in this example was controlled using a pH controller (FD-02 commercially available from Tokyo Garasu Kikai Co., Ltd.).

(Separating step and washing step)

Solid-liquid separation and washing were performed on the obtained aqueous dispersion having a pH of 2.5. Specifically, an operation of removing the supernatant by centrifugation (1000G, 10 minutes) and decantation, adding an amount of pure water equivalent to the removed amount, and thoroughly stirring with a spoon to achieve a uniform size was repeated 6 times, and finally, the above centrifugation and decantation were performed to obtain oxidized cellulose. The mass yield of the oxidized cellulose (amount of oxidized cellulose/amount of raw material cellulose$\times$100) was 63%.

(Salifying step)

Then, sodium hydroxide was added in an amount approximately equimolar to the amount of the introduced carboxy groups to return the carboxylic acid group from the proton form (-COO-H+) to the salt form (-COO-Na+), and thereby an aqueous dispersion having a pH of 7.5 was obtained. The content concentration of oxidized cellulose in the aqueous dispersion was 12 mass%. The degree of polymerization of the oxidized cellulose was 90.

(Fibrillation step)

Pure water was added to an aqueous dispersion having a pH of 7.5, the content concentration of oxidized cellulose was adjusted to 1 mass%, fibrillation was then performed with a homomixer (10,000 rpm, 10 minutes), and nanocellulose with an average fiber length of 200 nm and an average fiber width of 3 nm was obtained. When pure water was added to the obtained CNFs to prepare an aqueous dispersion with a solid content concentration of 0.1 mass%, the light transmittance (660 nm) thereof was 97%. A higher light transmittance value indicates better fibrillatability.

The light transmittance of CNF was a higher value than that of Comparative Example 1B to be described below, and one reason for this was to be speculated that, according to the protonating step, components that interfered with fibrillation of oxidized cellulose in the fibrillation step were removed.

[Example 2B]

Oxidized cellulose and nanocellulose were obtained in the same manner as in Example 1B except that, in the protonating step, an aqueous dispersion having a pH of 3.5 obtained by adding hydrochloric acid was used in place of an aqueous dispersion having a pH of 2.5, and in the salifying step, in place of obtaining an aqueous dispersion having a pH of 7.5, sodium hydroxide was added in an amount approximately equimolar to the amount of the introduced carboxy groups to obtain an aqueous dispersion having a pH of 7.3.

**The** mass yield of the obtained oxidized cellulose was 46%. The content concentration of oxidized cellulose after the salifying step was 11 mass%, and the degree of polymerization of the oxidized cellulose was 95.

In the obtained nanocellulose, the average fiber length was 210 nm, the average fiber width was 3 nm, and the light transmittance of an aqueous dispersion with a solid content concentration of 0.1 mass% was 95%.

[Example 3B]

Oxidized cellulose and nanocellulose were obtained in the same manner as in Example 1B except that, in place of repeating an operation of removing the supernatant by centrifugation (1000G, 10 minutes) and decantation, adding an amount of pure water equivalent to the removed amount, and thoroughly stirring with a spoon to achieve a uniform size 6 times, solid-liquid separation was performed by pressure filtration (0.2 MPa, and air-permeability of filter cloth of 0.3 cc/cm$^2$/sec), a liquid phase filtrate was removed, and the separated solid phase was washed with pure water having a pH of 6.8, and in the salifying step, in place of obtaining an aqueous dispersion having a pH of 7.5, sodium hydroxide in an amount approximately equimolar to the amount of the introduced carboxy groups was added to obtain an aqueous dispersion having a pH of 7.4.

During pressure filtration, clogging did not occur in the filter cloth, and subsequently, washing with pure water having a pH of 6.8 could be performed using the filter cloth. Slight white turbidity was confirmed in the filtrate when washed with pure water. Here, it was speculated that, the white turbidity occurred only in a part, and when washing with pure water having a pH of 6.8 was performed, carboxylic acid groups were dissociated from the proton form (-COO-H+) to the salt form (-COO-Na+), a part of the cellulose-based oxide was re-dispersed from the separated solid phase into the liquid phase, and white turbidity was caused in the filtrate.

The mass yield of the obtained oxidized cellulose was 67%. The content concentration of oxidized cellulose after the salifying step was 12 mass%, and the degree of polymerization of the oxidized cellulose was 92.

In the obtained nanocellulose, the average fiber length was 190 nm, the average fiber width was 3 nm, and the light transmittance of an aqueous dispersion with a solid content concentration of 0.1 mass% was 95%.

[Example 4B]

Oxidized cellulose and nanocellulose were obtained in the same manner as in Example 3B except that, in place of washing the separated solid phase with pure water having a pH of 6.8, the separated solid phase was washed with water whose pH was adjusted to 2.5 by adding hydrochloric acid to pure water.

Similar to the results of Example 3B, during pressure filtration, clogging did not occur in the filter cloth, and subsequently, washing with pure water could be performed using the filter cloth. However, no white turbidity was observed in the filtrate after washing.

The mass yield of the obtained oxidized cellulose was 69%. The content concentration of oxidized cellulose after the salifying step was 12 mass%, and the degree of polymerization of the oxidized cellulose was 91.

In the obtained nanocellulose, the average fiber length was 185 nm, the average fiber width was 3 nm, and the light transmittance of an aqueous dispersion with a solid content concentration of 0.1 mass% was 95%.

Compared to Example 3B, the mass yield of the oxidized cellulose was a higher value, and the reason for this was speculated to be that, when the separated solid phase was washed with water whose pH was adjusted to 2.5, carboxylic acid groups were not dissociated from the proton form (-COO-H+) to the salt form (-COO-Na+), and a part of the cellulose-based oxide was prevented from being re-dispersed from the separated solid phase into the liquid phase. Here, regarding the wastewater treatment for the filtrate, no occurrence of white turbidity in the filtrate was preferable in terms of treating the filtrate.

[Example 5B]

500 g of an aqueous sodium hypochlorite solution having a pH of 12.6 and an available chlorine concentration of 12 mass% was put into a jacketed glass container, and while stirring at 300 rpm with a stirrer (three-one motor, BL600 commercially available from Shinto Scientific Co., Ltd.) using three swept blades, heating was performed to 30°C, and 40 g of powdered pulp (KC FLOCK W-100GK, degree of crystallization: 38%, commercially available from Nippon Paper Industries Co., Ltd.) was then added as the cellulose raw material. After the cellulose raw material was supplied, while the temperature was maintained at 30°C, stirring was performed until the pH decreased to 10.3, and the pH during the reaction was then adjusted to 10.3 while adding a 25 mass% aqueous sodium hydroxide solution, and the mixture was stirred under the same conditions for a total of 7 hours after the cellulose raw material was added. After the reaction was completed, while checking the value of the oxidation-reduction potential, the remaining sodium hypochlorite was inactivated by adding an aqueous hydrogen peroxide solution. Then, hydrochloric acid was added to convert carboxy groups of the oxidized cellulose from the salt form (-COO-Na+) to the proton form (-COO-H+) to obtain an aqueous dispersion having a pH of 2.5. Solid-liquid separation was performed by pressure filtration at 0.2 MPa and washing with hydrochloric acid water having a pH of 2.5 was then performed. Sodium hydroxide was added to the obtained oxidized cellulose in the proton form to return the carboxylic acid group from the proton form (-COO-H+) to the salt form (-COO-Na+), and thereby a salt-form oxidized cellulose aqueous dispersion having a pH of 6.8 was obtained. The measured carboxy group content was 0.73 mmol/g, and the degree of polymerization was 100.

[Example 6B]

Oxidized cellulose and nanocellulose were obtained in the same manner as in Example 1B except that ashless cotton filter paper (a degree of crystallization of 59%, commercially available from Advantec Co., Ltd.) was used as the cellulose raw material.

The mass yield of the obtained oxidized cellulose was 65%. The content concentration of oxidized cellulose after the salifying step was 10 mass%, and the degree of polymerization of the oxidized cellulose was 90.

In the obtained nanocellulose, the average fiber length was 160 nm, the average fiber width was 10 nm, and the light transmittance of an aqueous dispersion with a solid content concentration of 0.1 mass% was 90%.

[Example 7B]

Example 7B was performed with reference to Patent Publication JP-A-2017-218470. In addition, referring to Patent Publication JP-A-2008-231258 which describes use of sea squirt husk as a raw material for the cellulose material, oxidized cellulose and nanocellulose were produced using sea squirt husk as the cellulose raw material.

The sea squirt husk was immersed in 0.2% NaOH at room temperature and then refined with a mixer. The sample was immersed in 5% NaOH at room temperature overnight, and then washed with water, and treated three times in a 0.3% aqueous sodium chlorite solution at 60°C for 2 hours to remove non-cellulose components. The treated product was sufficiently washed with water and then freeze-dried to obtain sea squirt husk cellulose.

Oxidized cellulose and nanocellulose were obtained in the same manner as in Example 1B except that the sea squirt husk cellulose was used as the cellulose raw material.

The mass yield of the obtained oxidized cellulose was 70%. The content concentration of oxidized cellulose after the salifying step was 8 mass%, and the degree of polymerization of the oxidized cellulose was 110.

In the obtained nanocellulose, the average fiber length was 230 nm, the average fiber width was 12 nm, and the light transmittance of an aqueous dispersion with a solid content concentration of 0.1 mass% was 85%.

[Comparative Example 1B]

Oxidized cellulose and nanocellulose were obtained in the same manner as in Example 1B except that the separating step was directly performed after the hypochlorous acid treating step without performing the protonating step, and in the salifying step, an aqueous dispersion having a pH of 7.4 was obtained by adding sodium hydroxide in excess of an amount equimolar to the amount of the introduced carboxy groups in place of obtaining an aqueous dispersion having a pH of 7.5.

The mass yield of the obtained oxidized cellulose was 18%. The content concentration of oxidized cellulose after the salifying step was 11 mass%, and the degree of polymerization of the oxidized cellulose was 98.

In the obtained nanocellulose, the average fiber length was 210 nm, the average fiber width was 3 nm, and the light transmittance of an aqueous dispersion with a solid content concentration of 0.1 mass% was 91%.

Compared to Example 1B, the mass yield of the oxidized cellulose was a significantly low value, and the reason for this was speculated to be that, in the separating step, when an operation of stirring with a spoon was performed, a part of the cellulose-based oxide was fibrillated into nanocellulose, and moved to the side of the supernatant to be removed.

[Reference Example 1B]

Oxidized cellulose and nanocellulose were obtained in the same manner as in Example 3B except that the separating step was directly performed after the hypochlorous acid treating step without performing the protonating step.

Unlike the results of Example 3B, during the pressure filtration, since clogging occurred in the filter cloth, the discharge velocity of the filtrate decreased considerably, and subsequently, it was not possible to wash with pure water using the filter cloth.

Industrial Applicability

According to the production method of the present invention, it is possible to provide nanocellulose used for various materials (for example, resins, fibers, rubber, etc.) and various applications (for example, food, cosmetics, medical products, paints, inks, etc.), and it has industrial applicability in various fields such as automobile members, machine parts, electrical appliances, electronic device, cosmetics, medical products, building materials, daily necessities, stationery and the like.

**Claims**

1. A method of producing an oxidized cellulose, which contains an oxide of a cellulose raw material by a hypochlorous acid or a salt thereof, is substantially free of N-oxyl compounds, and has a degree of polymerization of 600 or less, the method comprising

   a step of obtaining an oxidized cellulose by oxidizing a cellulose raw material using a hypochlorous acid or a salt thereof,
   wherein a viscosity of a slurry of the cellulose raw material having the same concentration as when the oxidation is performed, which is measured using a viscometer including an SPP rotor under measurement conditions of a rotational speed of 100 rpm and 30°C or 40°C, is in a range of 1,000 Pa·s or less.

2. The production method according to claim 1,
   wherein a concentration of the cellulose raw material with respect to a total amount of a reaction mixture is 35 mass% or less.

3. The production method according to claim 1 or 2,
   wherein the concentration of the cellulose raw material with respect to the total amount of the reaction mixture is more than 6.5 mass%.

4. The production method according to any one of claims 1 to 3,
   wherein an available chlorine concentration of the hypochlorous acid or the salt thereof in a reaction system is 6 mass% or more and 43 mass% or less.

5. The production method according to any one of claims 1 to 3,
   wherein an available chlorine concentration of the hypochlorous acid or the salt thereof in a reaction system is less than 14 mass%.

6. The production method according to any one of claims 1 to 5,
   wherein a reaction temperature for the oxidation is 30°C or higher.

7. The production method according to any one of claims 1 to 6,
   wherein a reaction time for the oxidation is 2 hours or longer.

8. The production method according to any one of claims 1 to 7,
   wherein pH of the reaction system is less than 11.

9. The production method according to any one of claims 1 to 8,
   wherein the viscosity is in a range of 30 Pa·s or less.

10. A method of producing a nanocellulose, the method comprising

a step of obtaining the nanocellulose through fibrillation after the step of oxidizing in the production method according to any one of claims 1 to 9.

11. A method of producing an oxidized cellulose, the method comprising

a step of obtaining the oxidized cellulose by solid-liquid separation of an oxide dispersion containing a cellulose-based oxide and a dispersion medium,
wherein pH of the oxide dispersion is 4.0 or less, and the oxide dispersion is substantially free of N-oxyl compounds.

12. The production method according to claim 11, further comprising
a step of obtaining the cellulose-based oxide by oxidizing a cellulose raw material using a hypochlorous acid or a salt thereof.

13. A method of producing an oxidized cellulose, the method comprising:

a step of obtaining a cellulose-based oxide by oxidizing a cellulose raw material using a hypochlorous acid or a salt thereof with a mass proportion of 0.2 or more with respect to the cellulose raw material; and
a step of obtaining the oxidized cellulose by solid-liquid separation of an oxide dispersion containing the cellulose-based oxide and a dispersion medium,
wherein pH of the oxide dispersion is 4.0 or less.

14. The production method according to claim 12 or 13, further comprising
a step of treating the hypochlorous acid or the salt thereof in the oxide dispersion.

15. The production method according to any one of claims 11 to 14, further comprising
a step of adding an acid and/or a cation ion exchange resin to prepare the oxide dispersion having pH of 4.0 or less.

16. The production method according to any one of claims 11 to 15, further comprising
a step of adding a base to adjust pH of an oxidized cellulose dispersion containing the oxidized cellulose and a dispersion medium to more than 4.0.

17. The production method according to any one of claims 11 to 16,
wherein the pH of the oxide dispersion is 2.5 or less.

18. The production method according to any one of claims 11 to 17,
wherein the step of obtaining the oxidized cellulose is a step of solid-liquid separation by filtering the oxide dispersion.

19. The production method according to any one of claims 11 to 18, further comprising
a step of washing the oxide dispersion or the oxidized cellulose with an acidic washing solution.

20. The production method according to any one of claims 11 to 19,
wherein a degree of polymerization of the oxidized cellulose is 600 or less.

21. Oxidized cellulose obtained by the production method according to any one of claims 11 to 20.

22. A method of producing a nanocellulose, the method comprising
a step of obtaining the nanocellulose by fibrillating the oxidized cellulose obtained by the production method according to any one of claims 11 to 20.

23. Nanocellulose obtained by the production method according to claim 22.

[FIG. 1]

```
                    ┌─────────────┐
                    │    START    │
                    └──────┬──────┘
                           │
                           ▼
                    ┌─────────────┐                    ┌─────────────┐
                    │  OXIDIZING  │                    │ SEPARATING  │◄───┐
                    │    STEP     │                    │    STEP     │    │
                    └──────┬──────┘                    └──────┬──────┘    │
                           │                                  │           │
                           ▼                                  ▼           │
                    ┌─────────────┐                    ┌─────────────┐    │
                    │  TREATING   │                    │   WASHING   │────┘
                    │    STEP     │                    │    STEP     │
                    └──────┬──────┘                    └──────┬──────┘
                           │                                  │
                           ▼                                  ▼
                    ┌─────────────┐                    ┌─────────────┐
                    │ PROTONATING │                    │  SALIFYING  │
                    │    STEP     │                    │    STEP     │
                    └─────────────┘                    └──────┬──────┘
                                                              │
                                                              ▼
                                                       ┌─────────────┐
                                                       │     END     │
                                                       └─────────────┘
```

## INTERNATIONAL SEARCH REPORT

| International application No. |
|---|
| **PCT/JP2021/047683** |

**A.   CLASSIFICATION OF SUBJECT MATTER**

*C08B 15/02*(2006.01)i; *C08B 15/04*(2006.01)i; *A61Q 1/00*(2006.01)i; *A61Q 19/00*(2006.01)i; *D21H 11/20*(2006.01)i; *A61K 8/73*(2006.01)i; *A61K 47/38*(2006.01)i
FI:   C08B15/04; D21H11/20; A61K47/38; A61K8/73; A61Q1/00; A61Q19/00; C08B15/02

According to International Patent Classification (IPC) or to both national classification and IPC

**B.   FIELDS SEARCHED**

Minimum documentation searched (classification system followed by classification symbols)

C08B15/02; C08B15/04; A61Q1/00; A61Q19/00; D21H11/20; A61K8/73; A61K47/38

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.   DOCUMENTS CONSIDERED TO BE RELEVANT**

| Category* | Citation of document, with indication, where appropriate, of the relevant passages | Relevant to claim No. |
|---|---|---|
| X | 荒木網男ほか, 酸化剤酸化法による微結晶セルロースの製造, 高分子化学, 1972, vol. 29, no. 239, pp. 652-656, entire text (column 2.1, tables 1, 2, 3, 5, etc.), non-official translation (ARAKI, Amio et al. Oxidizing Agent Production of Microcrystalline Cellulose by Oxidation Method. Polymer Chemistry.) entire text (column 2.1, tables 1, 2, 3, 5, etc.) | 1-10 |
| Y | | 1-10 |
| Y | WO 2020/027307 A1 (TOAGOSEI CO., LTD.) 06 February 2020 (2020-02-06) claims, examples | 1-10 |
| Y | WO 2020/184177 A1 (TOAGOSEI CO., LTD.) 17 September 2020 (2020-09-17) paragraph [0071] | 1-10 |
| Y | WO 2018/230354 A1 (TOAGOSEI CO., LTD.) 20 December 2018 (2018-12-20) examples 1-16 | 1-10 |
| Y | JP 2017-193814 A (KANTO DENKA KOGYO CO., LTD.) 26 October 2017 (2017-10-26) claims, examples 1-3 | 1-10 |

☑ Further documents are listed in the continuation of Box C.       ☑ See patent family annex.

| * Special categories of cited documents: | "T" later document published after the international filing date or priority date and not in conflict with the application but cited to understand the principle or theory underlying the invention |
|---|---|
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| **17 March 2022** | **05 April 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

| International application No. |
| --- |
| **PCT/JP2021/047683** |

**C.    DOCUMENTS CONSIDERED TO BE RELEVANT**

| Category* | Citation of document, with indication, where appropriate, of the relevant passages | Relevant to claim No. |
| --- | --- | --- |
| Y | JP 49-34989 A (ASAHI KASEI CORP.) 30 March 1974 (1974-03-30)<br>     examples 1, 2 | 1-10 |

Form PCT/ISA/210 (second sheet) (January 2015)

**INTERNATIONAL SEARCH REPORT**

International application No.

**PCT/JP2021/047683**

| Box No. III | Observations where unity of invention is lacking (Continuation of item 3 of first sheet) |
|---|---|

This International Searching Authority found multiple inventions in this international application, as follows:

The claims of the present application are classified into the following two inventions.

Invention 1: Claim 1 and claims 2-10 dependent thereon

Invention 2: claim 11 and claims 12 and 14-23 dependent thereon, and claim 13 and claims 14-23 dependent thereon

Claim 1 is considered to be the invention of a method for producing oxidized cellulose that contains an oxide of a cellulose raw material obtained using hypochlorous acid or a salt thereof, is substantially free from an N-oxyl compound and has a degree of polymerization or 600 or less, the method comprising a step for oxidizing the cellulose raw material with hypochlorous acid or a salt thereof to obtain the oxidized cellulose, characterized in that the oxidization is carried out under concentration conditions specified by certain parameters.

Meanwhile, the invention in claim 11 is considered to pertain to a method for producing oxidized cellulose, comprising a step for subjecting an oxide dispersion containing a cellulose oxide and a dispersion medium to solid-liquid separation to obtain the oxidized cellulose, wherein the dispersion is specified to have a specific pH value and to be substantially free from an N-oxyl compound.

When the inventions in claims 1 and 11 are compared, the inventions are common in that the products are oxidized cellulose substantially free from an N-oxyl compound. However, the starting material of the invention in claim 1 is a "cellulose raw material", while the starting material of the invention in claim 11 is an "oxide dispersion containing a cellulose oxide and a dispersion medium", and thus the inventions are different in this regard. In addition, the invention in claim 1 comprises a step for "oxidizing", while the invention in claim 11 does not comprise a step for "oxidizing", and the invention in claim 11 includes a step for "solid-liquid separation" which is not included in claim 1, and thus the inventions are completely different in terms of production steps. For the above reasons, the technical relevance between claims 1 and 11 is low in terms of invention of production methods. Further, although the technical feature shared between the inventions in claims 1 and 11 includes, as stated above, "oxidized cellulose substantially free from an N-oxyl compound", the technical feature is known in light of the contents in claims 1-6 respectively disclosing a method for producing oxidized cellulose without using N-oxyl compound, and thus is not a special technical feature.

Therefore, it cannot be said that claims 1 and 11 are in a group of inventions sharing a special technical feature, or are in a group of inventions so linked as to form a corresponding single general inventive concept.

Next, the invention in claim 13 pertains to a method for producing oxidized cellulose, comprising a step for performing oxidization using hypochlorous acid or a salt thereof at a mass ratio of 0.2 or more relative to a cellulose raw material to obtain a cellulose oxide and a step for subjecting an oxide dispersion containing the cellulose oxide and a dispersion medium to solid-liquid separation to obtain the oxidized cellulose, wherein the oxide dispersion has a pH of 4.0 or less.

When the inventions in claims 1 and 13 are compared, the invention in claim 13 is not specified by the parameters for concentration conditions of the cellulose raw material when the cellulose raw material is oxidized, and thus is different from the invention in claim 1 in terms of production steps. In addition, the invention in claim 13 is not specified by the degree of polymerization of the product, oxidized cellulose, or the content of N-oxyl compound, and thus is different from the invention in claim 1 in this regard. For the above reasons, the technical relevance between claims 1 and 13 is low in terms of invention of production methods. Further, although the technical feature shared between the inventions in claims 1 and 13 includes inclusion of a step for oxidizing a cellulose raw material using hypochlorous acid or a salt thereof, the technical feature is known in light of the contents in claims 1-6 respectively disclosing a method for producing oxidized cellulose using hypochlorous acid, and thus is not a special technical feature.

Therefore, it cannot be said that claims 1 and 13 are in a group of inventions sharing a special technical feature, or are in a group of inventions so linked as to form a corresponding single general inventive concept.

For the above reasons, the present application is considered to include two invention in total, namely invention 1 of claim 1 and claims 2-10 dependent thereon and invention 2 of claims 11 and 13 and claims 12 and 14-23 dependent thereon.

Form PCT/ISA/210 (continuation of first sheet) (January 2015)

## INTERNATIONAL SEARCH REPORT

| International application No. |
| --- |
| **PCT/JP2021/047683** |

**Box No. III    Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Claims 1-10**

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

## INTERNATIONAL SEARCH REPORT
### Information on patent family members

International application No.

**PCT/JP2021/047683**

| Patent document cited in search report | | | Publication date (day/month/year) | Patent family member(s) | | | Publication date (day/month/year) |
|---|---|---|---|---|---|---|---|
| WO | 2020/027307 | A1 | 06 February 2020 | EP | 3831856 | A1 | |
| | | | | claims, examples | | | |
| | | | | CN | 112513101 | A | |
| WO | 2020/184177 | A1 | 17 September 2020 | (Family: none) | | | |
| WO | 2018/230354 | A1 | 20 December 2018 | US | 2020/0270369 | A1 | |
| | | | | examples 1-16 | | | |
| | | | | EP | 3640264 | A1 | |
| | | | | CN | 110520448 | A | |
| JP | 2017-193814 | A | 26 October 2017 | (Family: none) | | | |
| JP | 49-34989 | A | 30 March 1974 | (Family: none) | | | |

Form PCT/ISA/210 (patent family annex) (January 2015)

34

**EP 4 269 448 A1**

**REFERENCES CITED IN THE DESCRIPTION**

**Patent documents cited in the description**

- WO 2018230354 A
- WO 2020027307 A
- JP 2017218470 A
- JP 2008231258 A

**Non-patent literature cited in the description**

- *Sustainable Chem. Eng.,* 2020, vol. 8 (48), 17800-17806